# EUROPEAN PATENT SPECIFICATION

(11) **EP 1 356 296 B1**
(45) Date of publication and mention of the grant of the patent: **20.04.2016**
(21) Application number: 01948711.5
(22) Date of filing: 25.06.2001
(51) Int. Cl.: B82Y 30/00, G01N 33/543, G01N 33/68

(54) **RAPID AND SENSITIVE DETECTION OF PROTEIN AGGREGATION**
SCHNELLE UND SENSITIVE FESTSTELLUNG VON PROTEINANHÄUFUNG
DETECTION RAPIDE ET SENSIBLE DE L'AGREGATION DES PROTEINES

(30) Priority: 23.06.2000 US 602689; 03.08.2000 US 631818
(43) Date of publication of application: 29.10.2003
(73) Proprietor: Minerva Biotechnologies Corporation, Waltham, MA 02451 (US)
(72) Inventor: BAMDAD, Cynthia, C., Newton, MA 02458 (US); BAMDAD, R., Shoshana, Newton, MA 02458 (US)
(74) Representative: Cowley, Catherine Mary
(86) International application number: PCT/US2001/020232
(87) International publication number: WO 2002/001230

(56) References cited:
- EP-A2- 0 489 465
- WO-A-00/43791
- WO-A-98/31839
- WO-A-98/37421
- WO-A-99/08695
- WO-A1-00/24941
- US-A- 5 620 850
- SIGAL G B ET AL: "SELF-ASSEMBLED MONOLAYER FOR THE BINDING AND STUDY OF HISTIDINE-TAGGED PROTEINS BY SURFACE PLASMON RESONANCE" ANALYTICAL CHEMISTRY, AMERICAN CHEMICAL SOCIETY. COLUMBUS, US, vol. 68, no. 3, 1996, pages 490-497, XP000916112 ISSN: 0003-2700
- TERZI E ET AL: "Interaction of Alzheimer beta-amyloid peptide(1-40) with lipid membranes." BIOCHEMISTRY. UNITED STATES 2 DEC 1997, vol. 36, no. 48, 2 December 1997 (1997-12-02), pages 14845-14852, XP002221485 ISSN: 0006-2960
- OTT P.: "Assessment of D-dimer in plasma: Diagnostic value in suspected deep venous thromobosis of the leg", ACTA MEDICA SCANDINAVICA, vol. 224, no. 3, 1988, pages 263-267,
- DEMPFLE C.E.: "Use of D-dimer assays in the diagnosis of venous thrombosis.", SEMINARS IN THROMBOSIS AND HEMOSTASIS, vol. 26, no. 6, 31 December 2000 (2000-12-31), pages 631-641, XP008085328,
- NAGATA M.: "Detection of fecal blood by colloidal gold agglutination using an anti-human hemoglobin monoclonal antibody.", JOURNAL OF IMMUNOLOGICAL METHODS, vol. 153, no. 1-2, 30 August 1992 (1992-08-30), pages 185-192,
- Carl S. Weisbecker ET AL: "Molecular Self-Assembly of Aliphatic Thiols on Gold Colloids", Langmuir, vol. 12, no. 16, 1 January 1996 (1996-01-01), pages 3763-3772, XP055041069, ISSN: 0743-7463, DOI: 10.1021/la950776r
- ELGHANIAN R ET AL: "Selective colorimetric detection of polynucleotides based on the distance-dependent optical properties of gold nanoparticles.", SCIENCE (NEW YORK, N.Y.) 22 AUG 1997, vol. 277, no. 5329, 22 August 1997 (1997-08-22), pages 1078-1081, ISSN: 0036-8075
- DEMERS L M ET AL: "A fluorescence-based method for determining the surface coverage and hybridization efficiency of thiol-capped oligonucleotides bound to gold thin films and nanoparticles.", ANALYTICAL CHEMISTRY 15 NOV 2000, vol. 72, no. 22, 15 November 2000 (2000-11-15), pages 5535-5541, ISSN: 0003-2700
- Harrell Sellers ET AL: "Structure and binding of alkanethiolates on gold and silver surfaces: implications for self-assembled monolayers", Journal of the American Chemical Society, vol. 115, no. 21, 1 October 1993 (1993-10-01), pages 9389-9401, XP055123187, ISSN: 0002-7863, DOI: 10.1021/ja00074a004

## Description

### Field of the Invention

This invention relates to methods, assays, and components for the rapid and sensitive detection and analysis of biomolecular interactions with particular applications to peptide aggregation associated with disease states such as neurodegenerative disease, and non-disease aggregation. Such methods and assays can be employed for clinical testing as well as to facilitate drug discovery by high-throughput drug screening.

### Background of the Invention

A variety of diseases are characterized by abnormal and toxic aggregation of biological molecules such as proteins and peptides, sometimes called protein condensation, tangle formation, fibril formation, plaque formation, etc. It would therefore be advantageous to identify inhibitors of these abnormal aggregation events. However, there are some diseases in which toxicity stems from the inhibition of a naturally occurring aggregation process. In these cases, it would be advantageous to identify molecules that restore the normal aggregation function.

An important class of disease associated with abnormal protein aggregation, and a major focus of the present invention, is neurodegenerative disease. Neurodegenerative diseases to which the invention is related include, without limitation (with their associated proteins involved in aggregation), Familial British Dementia (ABRI), Parkinson's Disease (α-synnuclein), Alzheimer's Disease (Aβ peptide), Finnish-type Familial Amyloidosis (Gelsoin), Huntington's Disease (Huntingtin), AD, Frontotemporal Dementia (Tau), Senile Systemic Amyloidosis (Transthyretin), Familial Amyloid Polyneuropathy (TTR), and Transmissible Spongiform Encephalopathie (PrP). Many neurodegenerative diseases have now been shown to be linked to and/or caused by plaque associated with aggregate formation in the brain that occurs as a result of aberrant aggregation of neuro peptides. Neurodegenerative diseases including Alzheimer's, Parkinson's, Gertsmann-Strausseler-Scheinker Syndrome, Fatal Familial Insomnia, Huntington's Chorea, Kuru, and Familial amyloid polyneuropathy and transmissible spongiform encephalopathies such as Creutzfeldt Jakob, Scrapie, and Bovine Spongiform Encephalopathy (BSE, Mad Cow), are characterized by ordered protein aggregates that form in the brain. Although the proteins that make up these aggregates share no sequence homology, or even conserved motif, the aggregates themselves share certain morphological features. *See* Lansbury, Proc. NatL Acad. Sci. USA, 96:3342 (1999). In these diseases, peptides related to the pathogenic state, which are normally soluble, undergo a conversion of their 3-dimensional structure via a mutation of the native peptide or a physical association with altered peptides, to an insoluble, ordered polymerized state that is characteristic of the abnormal protein deposits that are found in the brain in neurodegenerative diseases. This ordered polymerizing or aggregation may result from seeding with indogenous or exogenous agents or peptides.

For example, in Alzheimer's disease (AD), these aggregates are made up of β-amyloid protein that has undergone a conformational change, from soluble monomers, to insoluble, β-sheet oligomers. The concentration of these fibrils in the brain has been correlated to the progression of clinical disease. The growth profile of the characteristic fibrils is extremely non-linear, which could explain why its victims can appear asymptomatic for years, then suddenly undergo a rapid degeneration into dementia. Fibril formation *in vitro* is peptide concentration dependent. Short synthetic peptides, derived from the β-amyloid (Aβ) protein, can be made to form fibrils *in vitro* to mimic fibril formation that is characteristic of Alzheimer's disease. Aβ 1-42 (with an extended hydrophobic C-terminus) has been shown to form fibrils at a faster rate than Aβ 1-40. *See* J. Jarrett J et al, "The carboxy terminus of the β-amyloid protein is critical for the seeding of amyloid formation: implications for the pathogenesis of Alzheimer's disease," Biochemistry 32: 4693-4697 (1993). While Aβ 1-40 can form fibrils or aggregates on its own, solutions containing Aβ 1-40 undergo accelerated fibril formation if they are "mixed" with the less soluble 1-42 peptide or are "seeded" with pre-formed peptide fibrils. Although Aβ 1-40 is the predominant protein in neuritic plaque, these studies indicate that the rate of fibril formation may depend on the ratio of the concentration of 1-42 to 1-40. Consistent with these findings, all forms of early onset AD involve higher expression levels of the 1-42 peptide.

Atomic force microscopy (AFM) studies of *in vitro* fibril formation using the two most prevalent variants of Aβ, 1-42 and 1-40, have identified a metastable intermediate, termed the protofibril that occurs before fibril formation. *See* J. Harper et al, "Observation of metastable Aβ protofibrils by atomic force microscopy," Chem. and Biol 4: 119-125 (1997). The existence of these precursors to fibrils may explain the diffuse amyloid deposition observed in the brains of people pre-disposed to AD. The existence of a protofibril that is a toxic intermediate would explain several observed inconsistencies in vitro and in vivo that argued both for and against the Aβ fibril being the pathogenic agent of AD. Existing assays to test for neurodegenerative disease fibrils or fibril-forming species, or to screen for drugs suitable for treatment of neurodegenerative disease are Congo red and Thioflavin-T assays (Methods in Enzymology, Academic Press, 1999, Vol. 309, pgs. 274-287; 304-305) which typically cannot detect small aggregates or protofibrils. Specifically, they cannot detect aggregate or fibril-forming species at a concentration below about 100 µM. This is inadequate for detecting early stage disease, and for screening drugs suitable for use at early stage disease.

Drug candidates that act at a pre-symptomatic stage of the disease (when only small fibrils or aggregates are present) would have a greater efficiency in inhibiting plaque/fibril formation and preventing symptomatic disease. In order to do this, small fibril aggregates need to be efficiently detected. However, these aggregates are too small to be detected by nearly every detection method. Although they can be detected by AFM, this technique does not lend itself to clinical diagnostics or drug screening protocols. Therefore, at present, it has not been possible to screen for drugs that would act on the smaller fibrillar species. Additionally, screening for drugs to inhibit fibril formation at any stage has been severely limited.

The rate of aggregate and fibril formation is an extremely non-linear function of the concentration of converted or misfolded peptide, such as mutant neuro peptide or converted prion peptides, which are aggregate-forming or fibril-forming species. Once the concentration of the aberrant species reaches a critical concentration, the reaction rate proceeds too quickly to be affected by drug treatment. Therefore, for a drug to be effective at inhibiting plaque formation, thus making it a preventative therapeutic rather than a palliative one, it would necessarily have to act at an early stage. Current state of the art technology is not capable of detecting small aggregates or fibrils in a manner that is compatible with parallel drug screening methods or non-invasive diagnosis. This means that: 1) drugs to treat the early disease state cannot be readily identified; 2) pre-symptomatic patients cannot be identified; and 3) the effectiveness of potential drug candidates that exist, or will be identified in the future, cannot be accurately assessed.

Another drawback of existing technologies, for example, Congo Red and Thioflavin-T is that they require mechanical intervention during the process. That is, the process requires transfer of fluid from one container to another, and the like which disturbs the assay in a non-reproducible way accelerating fibril formation. Additionally, addition of the Congo Red and Thioflavin components quenches the reaction and stops the aggregation process, thus several time points cannot be taken of a single assay in solution.

One complication with the diagnosis of neurodegenerative disease is the fact that species capable of forming aggregates or fibrils characteristic of the disease may be present in extremely low concentration, yet at a concentration which, if detectable, could signify onset of the disease.

While information exists as to the process of neurodegenerative disease, a need exists for specific techniques for diagnosis and drug screening including simple, inexpensive, and reliable assays for detection of neurodegenerative disease, techniques for screening candidate drugs for treatment of neurodegenerative disease, and related components.

There are also many non-neurodegenerative diseases that involve aberrant protein aggregation. These diseases include but are not limited to the following: In multiple myeloma, antibody light chain aggregates to cause toxic effects. Waldenstroms Macroglobulinemia is a disease characterized by antibody heavy chain aggregation. A class of proteins, called cryoglobulins, precipitates at low temperatures, causing blockage of affected blood vessels. Disseminated intravascular coagulation (DIC) is a major cause of morbidity and mortality in people with severe systemic infections or autoimmune diseases, presumably because it depletes fibrin, resulting in an inhibition of blood clotting. Glanzmann's thrombasthenia is a bleeding disorder, characterized by abnormal platelet aggregation due to a defect in the integrin, alpha Iib beta 3¹. Abnormal fibronectin aggregation is characteristic of genetic human fibronectin-deposit glomerular disease². Sickle cell anemia is caused by mutant hemoglobin that aggregates rather than forming tetramers³. Abnormal protein aggregation has also been implicated as a toxic factor in stroke.

Of particular importance is abnormal protein aggregation involved in Type II Diabetes. Human islet amyloid polypeptide (hIAPP) is the major component of plaques found in the pancreatic islets of Langerhans in people who have type II diabetes mellitus⁴. Amino acids 20-29 have been shown to be responsible for this aggregation. The aggregation of hIAPP proceeds non-linearly as a function of time and can be accelerated by "seeding" the reaction with pre-formed aggregates, analogous to the case of beta-amyloid aggregation characteristic of Alzheimer's disease. However, unlike Alzheimer's disease, aggregation of hIAPP is independent of peptide concentration. An off-aggregation peptide micelle model has been suggested to explain this. Methods are disclosed that can be used to screen for drugs that inhibit this aggregation. For example, the hIAPP fragment that contains amino acids 20-29 are attached to colloids and added to solutions containing drug candidates. Solutions that contained drugs that inhibited aggregation remain pink while aggregation are characterized by a color change.

On the other hand, many disease states are characterized by a loss of a normal aggregation or multimerization process. A method to screen for therapeutics to restore this activity would also be useful. Blood clotting is accomplished by aggregation of the protein fibrin, which is a product of the precursor protein fibrinogen. Additionally, there are proteins that are not functional as monomers but must exist as dimers or tetramers in order to function. For many proteins, their biological function critically depends on their multimerization state. The aggregation assays described herein can readily be adapted to screen for molecules that either disrupt or enhance multimerization (a type of aggregation).

For example, the tumor suppressor protein p53, which is mutated in about 50% of all human cancers⁵, inhibits cell growth through activation of cell cycle arrest and apoptosis. Many aspects of p53's complicated function hinge on its multimerization state. Some research indicates that to be oncogenic, p53 needs to form tetramers⁶. Tetramerization is required for its DNA-binding⁷ and transcription activity. The tetramerization domain of p53 also contains a nuclear export signal (NES)⁸. It is believed that attenuation of p53 requires its export from the nucleus. Conversion of the tetramer to dimers and monomers exposes the NES. c-Abl, which is a growth suppressor binds to the C-terminus of p53 only if it is a tetramer and this stimulates DNA binding⁹. There may be therapeutic value in identifying molecules that stimulate p53 tetramerization as well as those that disrupt it.

### Summary Of The Invention

In a first aspect of the invention, there is provided (insert language of Claim 1).

In a second aspect of the invention, there is provided (insert language of Claim 18).

Preferred embodiments of the invention in its various aspects are as described below or as defined in the subclaims.

### Brief Description of the Drawings

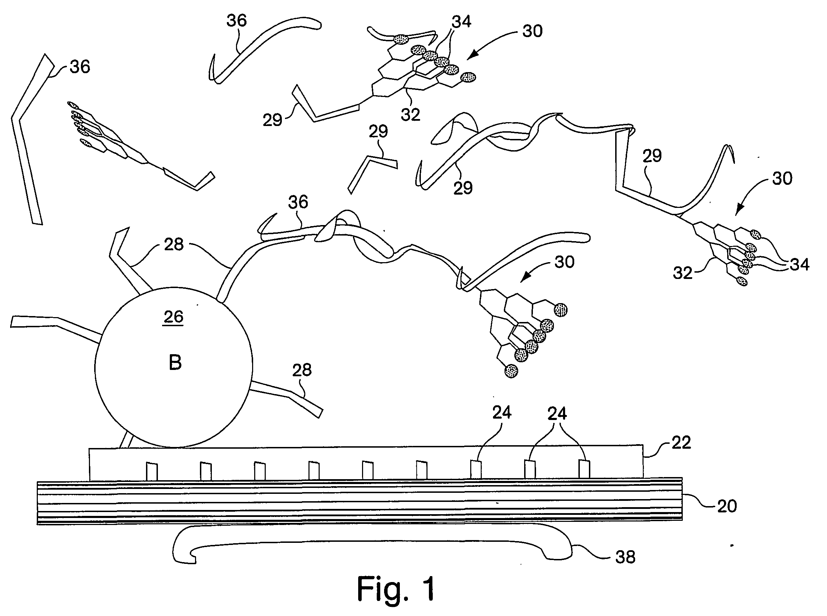

Figure 1 depicts peptides capable of participating in fibril or plaque formation attached to metal-containing compounds, such as ferrocene, via dendrimers or polymers. These derivatized peptides become associated with magnetic particles presenting similar peptides through their simultaneous incorporation into aggregates associated with neurodegenerative disease. After recruitment to a SAM-coated electrode via a magnet, the presence of the aggregates is determined by detecting the presence of incorporated electroactive signaling moieties, like ferrocene, using techniques such as cyclic voltammetry (CV) or alternating current voltammetry (ACV).
Figure 2 depicts peptides capable of participating in aggregate formation and a metal complex, such as a ferrocene derivative, attached to a colloid particle. These peptides become associated with magnetic particles bearing similar peptides when both are incorporated into aggregates of abnormal versions of these prion-type (or fibril forming) peptides in the solution. After recruitment to a SAM-coated electrode via a magnet, the presence of the aggregates is electronically detected.
Figure 3 is a bar graph showing the detection of fibril formation by spectrophotometric means.
Figure 4 is a photocopy of a photograph of a negative control in one assay.
Figure 5 is a photocopy of a photograph of a forty-fold magnification of aggregates, easily visibly identifiable to the unaided human eye, formed of an assay.
Figure 6 is a photocopy of a photograph of small aggregates visible under a microscope, formed in an assay of the invention from 50 picomolar Aβ fibrils (figs. 4-6 all are magnified 40x).
Figure 7A is a photocopy of a photograph of a 40x magnified large colloid aggregation, readily visibly identifiable to the unaided human eye, formed in accordance with another assay.
Figure 7B is a photocopy of a photograph of the negative control of the assay of Figure 7A.
Figure 8 is a color copy of a photograph of an ELISA plate showing the results of a drug screening assay.
Figure 9 is an overlay of four ACVs showing electronic detection of a fibril in another assay.
Figure 10 shows the results of a light-scattering fibril assay.
Figure 11 is the negative control of the light-scattering experiment of Figure 10.
Figure 12 is a graph showing drug activity as a function of time in a neurodegenerative disease assay, correlating drug activity to aggregate size.
Figure 13 shows results, in color, of an assay in which colloids aggregate indicative of a binding interaction, as compared to a control.
Figure 14 shows color change, from pink to blue (indicating colloid aggregation), resulting from increased Aβ concentration in a neurodegenerative disease assay, or the passage of time in an assay with constant concentration.

### Detailed Description of the Invention

International patent application serial number PCT/US00/01997, filed 01/25/00 by Bamdad et al., entitled "Rapid and Sensitive Detection of Aberrant Protein Aggregation in Neurodegenerative Diseases", International patent application serial number PCT/US00/01504, filed 01/21/00 by Bamdad, et al, entitled "Interaction of Colloid-Immobilized Species with Species on Non-Colloidal Structures", and commonly-owned, copending U.S. patent application filed on even date herewith by Bamdad et al., entitled "Interaction of Colloid-Immobilized Species with Species on Non-Colloidal Structures" are referred to.

### Definitions

"Small molecule", as used herein, means a molecule less than 5 kiloDalton, more typically less than 1 kiloDalton. As used herein, "small molecule" excludes proteins.

The term "aggregates" refers to complexes of homologous molecules (e.g. fibrils) as well as mixtures of molecules (e.g. interacting molecules such as binding partners, peptides, proteins, nucleic acids or complexes of these that promote the formation of aggregates, including complexes characterized by specific interactions). "Aggregates" can be associated with disease states such as neurodegenerative disease and other disease, and can be associated with non-disease states. Aggregates associated with neurodegenerative disease typically involve aggregation of proteinaceous molecules such as tangles or plaques characteristic and are associated with, i.e. may cause, neurodegenerative disease. Neurodegenerative disease aggregates typically are complexes of homologous molecules, but may include mixtures. Non-neurodegenerative disease aggregates include those associated with type II diabetes, sickle-cell anemia, which diseases involve aggregates similar to those of neurodegenerative disease, as well as other non-neurodegenerative disease aggregates such as myeloma, which involves light or heavy chain IgG agglomeration. Aggregates that are not associated with disease include those associated with, e.g., blood clotting (fibrinogen, plasminogen aggregates, and related factors), aggregates that act as tumor suppressors such as P53 aggregates, and the like. Those of ordinary skill in the art understand aggregates as they relate to neurodegenerative disease, non-neurodegenerative disease, and non-disease physiological processes. Aggregates associated with each of these processes are distinguishable from each other by those of ordinary skill in the art. "Aggregates", as used herein, encompasses fibrils. All fibrils are aggregates but not all aggregates are fibrils. Aggregates are typically of such a size (by virtue of the colloid interaction) that they can be visualized by eye, under a microscope, by light scattering, or by color changes.

While "aggregates" is used to refer to collections of molecules or fragments involved with disease and non-disease processes described herein (aggregates is defined below), it is to be understood that fibrils are types of aggregates involved in certain disease processes. Where "fibrils" are described herein, it is to be understood that they are aggregates.

The phrase "render the aggregate visible" refers to techniques whereby a non-visible aggregate is made visible to the eye, visible under a microscope, or visible by absorbance. A variety of approaches are contemplated, including but not limited to attaching or decorating aggregate with particles with specific absorption characteristics, such as gold colloids, particles carrying color-generating entities, etc.

The term "candidate drug" as used herein, refers to any medicinal substance used in humans, animals, or plants. Encompassed within this definition are compound analogs, naturally occurring, synthetic and recombinant pharmaceuticals, hormones, antimicrobials, neurotransmitters, etc. This includes any substance or precursor (whether naturally occurring, synthetic or recombinant) which is to be evaluated for use as a drug for treatment of neurodegenerative disease, or other disease characterized by aberrant aggregation, or prevention thereof. Evaluation typically takes place through activity in an assay, such as the screening assays described herein.

A variety of types of particles can be used. For example, "fluid suspendable particle" means a particle that can be made to stay in suspension in a fluid in which it is used for purposes as described (typically an aqueous solution) by itself, or can be maintained in solution by application of a magnetic field, an electromagnetic field, agitation such as stirring, shaking, vibrating, sonicating, centrifuging, vortexing, or the like. A "magnetically suspendable" particle is one that can be maintained in suspension in a fluid via application of a magnetic field. An electromagnetically-suspendable particle is one that can be maintained in suspension in a fluid by application of an electromagnetic field (e.g., a particle carrying a charge, or a particle modified to carry a charge). A "self-suspendable particle" is a particle that is of low enough size and/or mass that it will remain in suspension in a fluid in which it is used (typically an aqueous solution), without assistance of for example a magnetic field, for at least 1 hour. Other self-suspendable particles will remain in suspension, without assistance, for 5 hours, 1 day, 1 week, or even 1 month.

As used herein, a "binding species capable of binding an aggregate-forming species" includes any species such as a protein, peptide, sequence of either, antibody, Congo red, Thioflavin-T, or any species capable of the binding so described. In some cases, this binding can be site-specific, such as in the case of antibodies or P53. In other cases it can be non-specific. In the case of proteins or peptides, the binding typically involves non-specific hydrophobic interaction. In the case of neurodegenerative disease, β-sheet/ β-sheet interactions typically are involved. Binding species also can include peptides, fragments, or whole proteins that are homologous to naturally-occurring disease aggregate-forming species.

"Proteins" and "peptides" are well-known terms in the art, and are not precisely defined in the art in terms of the number of amino acids that each includes. As used herein, these terms are given their ordinary meaning in the art. Generally, peptides are amino acid sequences of less than about 100 amino acids in length, but can include sequences of up to 300 amino acids. Proteins generally are considered to be molecules of at least 100 amino acids.

As used herein, a "metal binding tag" refers to a group of molecules that can become fastened to a metal that is coordinated by a chelate. Suitable groups of such molecules include amino acid sequences, typically from about 2 to about 10 amino acid residues. These include, but are not limited to, histidines and cysteines ("polyamino acid tags"). Such binding tags, when they include histidine, can be referred to as a "poly-histidine tract" or "histidine tag" or "HIS-tag", and can be present at either the amino- or carboxy-terminus, or at any exposed region, of a peptide or protein or nucleic acid. A poly-histidine tract of six to ten residues is preferred. The poly-histidine tract is also defined functionally as being a number of consecutive histidine residues added to a protein of interest which allows the affinity purification of the resulting protein on a metal chelate column, or the identification of a protein terminus through the interaction with another molecule (e.g. an antibody reactive with the HIS-tag).

"Affinity tag" is given its ordinary meaning in the art. Affinity tags include, for example, metal binding tags, GST (in GST/glutathione binding clip), and streptavidin (in biotin/streptavidin binding). At various locations herein specific affinity tags are described in connection with binding interactions. It is to be understood that the present description, when employing an affinity tag, may involve selection of any of the affinity tags described herein.

As used herein, "chelate coordinating a metal" or metal coordinated by a chelate, refers to a metal coordinated by a chelating agent that does not fill all available coordination sites on the metal, leaving some coordination sites available for binding via a metal binding tag.

"Signaling entity" means an entity that is capable of indicating its existence in a particular sample or at a particular location. Signaling entities can be those that are identifiable by the unaided human eye, those that may be invisible in isolation but may be detectable by the unaided human eye if in sufficient quantity (e.g., colloid particles), entities that absorb or emit electromagnetic radiation at a level or within a wavelength range such that they can be readily detected visibly (unaided or with a microscope including an electron microscope or the like), or spectroscopically, entities that can be detected electronically or electrochemically, such as redox-active molecules exhibiting a characteristic oxidation/reduction pattern upon exposure to appropriate activation energy ("electronic signaling entities"), or the like. Examples include dyes, pigments, electroactive molecules such as redox-active molecules, fluorescent moieties (including, by definition, phosphorescent moieties), up-regulating phosphors, chemiluminescent entities, electrochemiluminescent entities, or enzyme-linked signaling moieties including horse radish peroxidase and alkaline phosphatase. "Precursors of signaling entities" are entities that by themselves may not have signaling capability but, upon chemical, electrochemical, electrical, magnetic, or physical interaction with another species, become signaling entities. An example includes a chromophore having the ability to emit radiation within a particular, detectable wavelength only upon chemical interaction with another molecule. Precursors of signaling entities are distinguishable from, but are included within the definition of, "signaling entities" as used herein.

As used herein, "fastened to or adapted to be fastened", in the context of a species relative to another species or to a surface of an article, means that the species is chemically or biochemically linked via covalent attachment, attachment via specific biological binding (e.g., biotin/streptavidin), coordinative bonding such as chelate/metal binding, or the like. For example, "fastened" in this context includes multiple chemical linkages, multiple chemical/biological linkages, etc., including, but not limited to, a binding species such as a peptide synthesized on a polystyrene bead, a binding species specifically biologically coupled to an antibody which is bound to a protein such as protein A, which is covalently attached to a bead, a binding species that forms a part (via genetic engineering) of a molecule such as GST or Phage, which in turn is specifically biologically bound to a binding partner covalently fastened to a surface (e.g., glutathione in the case of GST), etc. As another example, a moiety covalently linked to a thiol is adapted to be fastened to a gold surface since thiols bind gold covalently. Similarly, a species carrying a metal binding tag is adapted to be fastened to a surface that carries a molecule covalently attached to the surface (such as thiol/gold binding) which molecule also presents a chelate coordinating a metal. A species also is adapted to be fastened to a surface if a surface carries a particular nucleotide sequence, and the species includes a complementary nucleotide sequence.

"Covalently fastened" means fastened via nothing other than one or more covalent bonds. E.g. a species that is covalently coupled, via EDC/NHS chemistry, to a carboxylate-presenting alkyl thiol which is in turn fastened to a gold surface, is covalently fastened to that surface.

"Specifically fastened" or "adapted to be specifically fastened" means a species is chemically or biochemically linked to another specimen or to a surface as described above with respect to the definition of "fastened to or adapted to be fastened", but excluding all non-specific binding.

"Non-specific binding", as used herein, is given its ordinary meaning in the field of biochemistry.

"Colloids", as used herein, means nanoparticles, i.e. very small, self-suspendable or fluid-suspendable particles including those made of material that is, e.g., inorganic or organic, polymeric, ceramic, semiconductor, metallic (e.g. gold), non-metallic, crystalline, amorphous, or a combination. Typically, colloid particles used in accordance with the invention are of less than 250 nm cross section in any dimension, more typically less than 100 nm cross section in any dimension, and in most cases are of about 2-30 nm cross section. One class of colloids suitable for use in the invention is 10-30 nm in cross section, and another about 2-10 nm in cross section. As used herein this term includes the definition commonly used in the field of biochemistry.

A "moiety that can coordinate a metal", a used herein, means any molecule that can occupy at least two coordination sites on a metal atom, such as a metal binding tag or a chelate.

As used herein, a component that is "immobilized relative to" another component either is fastened to the other component or is indirectly fastened to the other component, e.g., by being fastened to a third component to which the other component also is fastened, or otherwise is translationally associated with the other component. For example, a signaling entity is immobilized with respect to a binding species if the signaling entity is fastened to the binding species, is fastened to a colloid particle to which the binding species is fastened, is fastened to a dendrimer or polymer to which the binding species is fastened, etc. A colloid particle is immobilized relative to another colloid particle if a species fastened to the surface of the first colloid particle attaches to an entity, and a species on the surface of the second colloid particle attaches to the same entity, where the entity can be a single entity, a complex entity of multiple species, a cell, another particle, etc.

"Aggregate-forming species", as used herein, means biological species associated with disease or non-disease processes involving aggregates, as defined above, having sufficient binding capacity to bind to other molecules associated with the disease or non-disease process (including like molecules), to form fibrils or aggregates characteristic of the process. In some disease processes such as neurodegenerative disease, such aggregate-forming species typically are characterized by a change in molecule conformation, relative to sequence-homologous, healthy, counterparts, allowing them to bind more readily to like or similar molecules. In some cases, such as in neurodegenerative disease, type-II diabetes, and myeloma, such aggregate-forming species have the capability to convert binding species from non-aggregate-forming conformation into aggregate-forming conformation. Protofibrils formed from neurodegenerative disease aggregate-forming species have been reported to be a precursor of, or an early from of, neurodegenerative disease associated aggregates. Protofibrils are included in the definition of aggregate-forming species as used herein. Similar to the term "aggregates", those of ordinary skill in the art understand the meaning of the term "aggregate forming species" as it is applied to neurodegenerative disease aggregate-forming species, non-neurodegenerative disease aggregate-forming species, and non-disease aggregate-forming species.

"Diverse biological species" means different animals, such as mouse and hamster, mouse and goat, etc.

The term "sample" refers to any cell, tissue, or fluid from a biological source (a "biological sample", or any other medium, biological or non-biological, that can advantageously be evaluated including, but not limited to, a biological sample drawn from a human patient, a sample drawn from an animal, a sample drawn from food designed for human consumption, a sample including food designed for animal consumption such as livestock feed, milk, an organ donation sample, a sample of blood destined for a blood supply, a sample from a water supply, or the like. One example of a sample is a sample drawn from a human or animal to whom a candidate drug has been given to determine the efficacy of the drug.

A "sample suspected of containing" a particular component means a sample with respect to which the content of the component is unknown. For example, a fluid sample from a human suspected of having a disease, such as a neurodegenerative disease or a non-neurodegenerative disease, but not known to have the disease, defines a sample suspected of containing neurodegenerative disease aggregate-forming species. "Sample" in this context includes naturally-occurring samples, such as physiological samples from humans or other animals, samples from food, livestock feed, etc., as well as "structurally predetermined samples", which are defined herein to mean samples, the chemical or biological sequence or structure of which is a predetermined structure used in an assay designed to test whether the structure is associated with a particular process such as a neurodegenerative disease. For example, a "structurally predetermined sample" includes a peptide sequence, random peptide sequence in a phage display library, and the like. Typical samples taken from humans or other animals include cells, blood, urine, ocular fluid, saliva, cerebro-spinal fluid, fluid or other samples from tonsils, lymph nodes, needle biopsies, etc.

As used herein, a "metal binding tag" refers to a group of molecules that can become fastened to a metal that is coordinated by a chelate. Suitable groups of such molecules include amino acid sequences, typically from about 2 to about 10 amino acid residues. These include, but are not limited to, histidines and cysteines ("polyamino acid tags"). Such binding tags, when they include histidine, can be referred to as a "poly-histidine tract" or "histidine tag" or "HIS-tag", and can be present at either the amino- or carboxy-terminus, or at any exposed region, of a peptide or protein or nucleic acid. A poly-histidine tract of six to ten residues is preferred for use in the invention. The poly-histidine tract is also defined functionally as being a number of consecutive histidine residues added to a protein of interest which allows the affinity purification of the resulting protein on a metal chelate column, or the identification of a protein terminus through the interaction with another molecule (e.g. an antibody reactive with the HIS-tag). "Affinity tag" is given its ordinary meaning in the art. Affinity tags, include, by definition, metal binding tags.

As used herein, "metal binding tag/metal/chelate linkage" defines a linkage between first and second species in which a first species is immobilized relative to a metal binding tag and a second species is immobilized relative to a chelate, where the chelate coordinates a metal to which the metal binding tag is also coordinated. U.S. Patent No. 5,620,850 of Bamdad, et al. describes exemplary linkages.

The term "biological binding" refers to the interaction between a corresponding pair of molecules that exhibit mutual affinity or binding capacity, typically specific or non-specific binding or interaction, including biochemical, physiological, and/or pharmaceutical interactions. Biological binding defines a type of interaction that occurs between pairs of molecules including proteins, nucleic acids, glycoproteins, carbohydrates, hormones and the like. Specific examples include antibody/antigen, antibody/hapten, enzyme/substrate, enzyme/inhibitor, enzyme/cofactor, binding protein/substrate, carrier protein/substrate, lectin/carbohydrate, receptor/hormone, receptor/effector, complementary strands of nucleic acid, protein/nucleic acid repressor/inducer, ligand/cell surface receptor, virus/ligand, etc.

The term "binding partner" refers to a molecule that can undergo binding with a particular molecule. Biological binding partners are examples. For example, Protein A is a binding partner of the biological molecule IgG, and vice versa.

The term "determining" refers to quantitative or qualitative analysis of a species via, for example, spectroscopy, ellipsometry, piezoelectric measurement, immunoassay, electrochemical measurement, and the like. "Determining" also means detecting or quantifying interaction between species, e.g. detection of binding between two species.

The term "self-assembled monolayer" (SAM) refers to a relatively ordered assembly of molecules spontaneously chemisorbed on a surface, in which the molecules are oriented approximately parallel to each other and roughly perpendicular to the surface. Each of the molecules includes a functional group that adheres to the surface, and a portion that interacts with neighboring molecules in the monolayer to form the relatively ordered array. See Laibinis, P. E.; Hickman, J.; Wrighton, M. S.; Whitesides, G. M. Science 245, 845 (1989), Bain, C.; Evall, J.; Whitesides, G. M. J. Am. Chem. Soc. 111, 7155-7164 (1989), Bain, C.; Whitesides, G. M. J. Am. Chem. Soc. 111, 7164-7175 (1989),. Preferably, self-assembled monolayers (SAMs) on surfaces are used, such as surfaces of colloid particles, and articles such as colloid particles having surfaces coated with SAMs. Preferably, SAMs formed completely of synthetic molecules completely cover a surface or a region of a surface, e.g. completely cover the surface of a colloid particle. "Synthetic molecules", in this context, means a molecule that is not naturally occurring, rather, one synthesized under the direction of human or human-created or human-directed control. "Completely cover" in this context, means that there is no portion of the surface or region that directly contacts a protein, antibody, or other species that prevents complete, direct coverage with the SAM. I.e. preferably, the surface or region includes, across its entirety, a SAM consisting completely of non-naturally-occurring molecules (i.e. synthetic molecules). The SAM can be made up completely of SAM-forming species that form close-packed SAMs at surfaces, or these species in combination with molecular wires or other species able to promote electronic communication through the SAM (including defect-promoting species able to participate in a SAM), or other species able to participate in a SAM, and any combination of these. Preferably, all of the species that participate in the SAM include a functionality that binds, optionally covalently, to the surface, such as a thiol which will bind to a gold surface covalently. A self-assembled monolayer on a surface can be comprised of a mixture of species (e.g. thiol species when gold is the surface) that can present (expose) essentially any chemical or biological functionality. For example, they can include tri-ethylene glycol-terminated species (e.g. tri-ethylene glycol-terminated thiols) to resist non-specific adsorption, and other species (e.g. thiols) terminating in a binding partner of an affinity tag, e.g. terminating in a chelate that can coordinate a metal such as nitrilotriacetic acid which, when in complex with nickel atoms, captures a metal binding tagged-species such as a histidine-tagged binding species. The present description provides a method for rigorously controlling the concentration of essentially any chemical or biological species presented on a colloid surface or any other surface. The self-assembled monolayer is formed on gold colloid particles.

The term "self-assembled mixed monolayer" refers to a heterogeneous self-assembled monolayer, that is, one made up of a relatively ordered assembly of at least two different molecules.

"Molecular wires" as used herein, means wires that enhance the ability for a fluid encountering a SAM-coated electrode to communicate electrically with the electrode. This includes conductive molecules or, as mentioned above and exemplified more fully below, molecules that can cause defects in the SAM allowing communication with the electrode. A non-limiting list of additional molecular wires includes 2-mercaptopyridine, 2-mercaptobenzothiazole, dithiothreitol, 1, 2-benzenedithiol, 1, 2-benzenedimethanethiol, benzene-ethanethiol, and 2-mercaptoethylether. Conductivity of a monolayer can also be enhanced by the addition of molecules that promote conductivity in the plane of the electrode. Conducting SAMs can be composed of, but are not limited to: 1) poly (ethynylphenyl) chains terminated with a sulfur; 2) an alkyl thiol terminated with a benzene ring; 3) an alkyl thiol terminated with a DNA base; 4) any sulfur terminated species that packs poorly into a monolayer; 5) all of the above plus or minus alkyl thiol spacer molecules terminated with either ethylene glycol units or methyl groups to inhibit non specific adsorption. Thiols are described because of their affinity for gold in ready formation of a SAM. Other molecules can be substituted for thiols as known in the art from U.S. Patent No. 5,620,820, and other references. Molecular wires typically, because of their bulk or other conformation, creates defects in an otherwise relatively tightly-packed SAM to prevent the SAM from tightly sealing the surface against fluids to which it is exposed. The molecular wire causes disruption of the tightly-packed self-assembled structure, thereby defining defects that allow fluid to which the surface is exposed to communicate electrically with the surface. In this context, the fluid communicates electrically with the surface by contacting the surface or coming in close enough proximity to the surface that electronic communication via tunneling or the like, can occur.

Referring now to Figure 1, one arrangement is illustrated schematically. Shown in Figure 1 is an article 20, specifically an electrode including a SAM 22 on a surface thereof. SAM 22 includes "molecular wires" 24, where it is desirable that SAM 22 be relatively electrically conductive. Typically, a mixed SAM is formed of a relatively non-conducting species such as an alkyl thiol optionally terminating in a non-specific binding inhibitor such as polyethylene glycol, mixed with molecular wires. The selection of surfaces and functional groups for binding self-assembling monolayer molecules to surfaces is a well-known art. See U.S. Patent Nos. 5,512,131 and 5,620,850, and International Patent Publication no. WO 96/29629 for this and other teachings.

A particle 26, specifically a magnetic bead, is provided along with a plurality of binding species, capable of binding an aggregate-forming species including that associated with neurodegenerative disease or non-neurodegenerative disease, or other aggregate-forming species, immobilized relative to or adapted to be immobilized relative to the bead. A plurality of binding species 28 also are provided fastened or fastenable to signaling entities 30 which, comprise dendrimers 32, each carrying a plurality of individual signaling entities 34. A plurality of target molecules 36, which are neurodegenerative disease or non-neurodegenerative disease aggregate-forming or other fibril-forming species, are introduced by, for example, being included in a physiological sample suspected of containing the molecules. Binding species 28 bind to aggregate-forming or fibril-forming species 36 to define a linkage including bead 26 with attached binding species, signaling entity 30 with attached binding species, and aggregate or fibril-forming species 36 immobilized with respect to each. This arrangement can be drawn to a surface of electrode 20 via a magnet 38 under the electrode and, where signaling entities 34 are electronic or electro-active (redox active) signaling entities, their proximity near electrode 20 can be detected indicating presence of the aggregate or fibril-forming species 36 in the sample.

Specifically, electronic signaling entity 34 can be a redox-active molecule such as a metallocene, specifically ferrocene (i.e. a ferrocene derivative). Proximity of a ferrocene as signaling entity 34 near electrode 20 can be determined by a cyclic voltammetric technique such as alternating current voltammetry (ACV) or via a device employing charge counting.

Binding species 28 need not be fastened to signaling entity 30 and/or bead 26 initially, but a mixture can be provided including a sample suspected of containing species 36, binding species 28, signaling entity 30, and bead 26, where binding species 28 is adapted to be fastened to signaling entity 30 and/or bead 26 if not already fastened. In this arrangement, species 28 can be adapted to be fastened to signaling entity 30 and/or bead 26 by including a chemical or biological binding partner of a molecule fastened to signaling entity 30 or bead 26. Bead 26, if a polymeric bead, can include a variety of covalently-attached linking molecules. Similarly, entity 30 can be modified in this way. Bead 26 also can be coated with a surface layer of a material that facilitates formation of a SAM thereon, such as gold, and a SAM forming molecule (such as a thiol in the case of the gold surface) carrying a linking molecule can be formed thereon. Linkages between a linking molecule on bead 26 or signaling entity 30 and binding species 28 can include an affinity tag such as metal binding tag/metal/chelate linkages, complementary nucleic acid sequences, biotin/streptavidin, etc. In an arrangement where a metal binding tag/metal/chelate linkage is used, a chelate can form part of a SAM on bead 26, and can be covalently attached to signaling entity 30. The chelate coordinates a metal, but leaves at least two open coordinate sites on the metal. A metal binding tag such as a polyamino acid tag can be incorporated into binding species 36, giving species 36 the ability to fasten to the bead or signaling entity by coordination of the metal binding tag to the metal. Examples of suitable chelates include nitrilotriacetic acid, 2,2'-bis(salicylideneamino)-6,6'-demethyldiphenyl, or 1,8-bis(a-pyridyl)-3,6-dithiaoctane. In an alternate immobilization technique, binding species 36 can carry a terminal cysteine and fasten thereby to a gold surface of bead 26.

A variety of binding species 36 can be selected. The binding species can be a peptide, protein, sequence from a protein, small molecule such as Congo red or Thioflavin-T, or sequence homologous to sequence derived from aggregate-forming species, RNA, DNA, nucleoside derivatives, or antibody to the aggregate-forming or fibril-forming species 36.

A variety of surfaces can form components of kits or systems or participate in methods. Surfaces such as surfaces of beads, colloids, electrodes, ELISA plates, other multi-well plates, and the like can be used.

In many techniques it is desirable to prevent any binding species introduced into an assay from participating in aggregate formation. In such a case, aggregate-binding, but aggregate-formation-resistant binding species can be used. Aggregate-formation-resistant species can easily be selected by those of ordinary skill in the art from among species such as antibodies, fragments of proteins or peptides, etc. that will bind but not form aggregates.

The surface of the article, whether it be an electrode, particle, bead, colloid, or the like, preferably has a chemical functionality that substantially inhibits non-specific binding of aggregate-forming species. This can be accomplished by forming a SAM on the surface for presentation of a desired functionality for the assay, the SAM being a mixed SAM including other non-specific-binding inhibitors. NSB inhibitors include polyethylene glycol-terminated SAM-forming species.

Preferably, a SAM coating the colloid inhibits colloid/colloid self-aggregation. This can be accomplished by incorporating a sufficient amount of SAM-forming species that are charged in addition to species that resist NSB. Charged moieties and/or carboxy-terminated species should be present in an amount of about 10 percent or more of the SAM, or preferably at least about 30 percent or at least about 45 percent of the SAM. These species can also be present in higher percentages such as, for example, 60 percent, 80 percent, 90 percent, or even 95 percent.

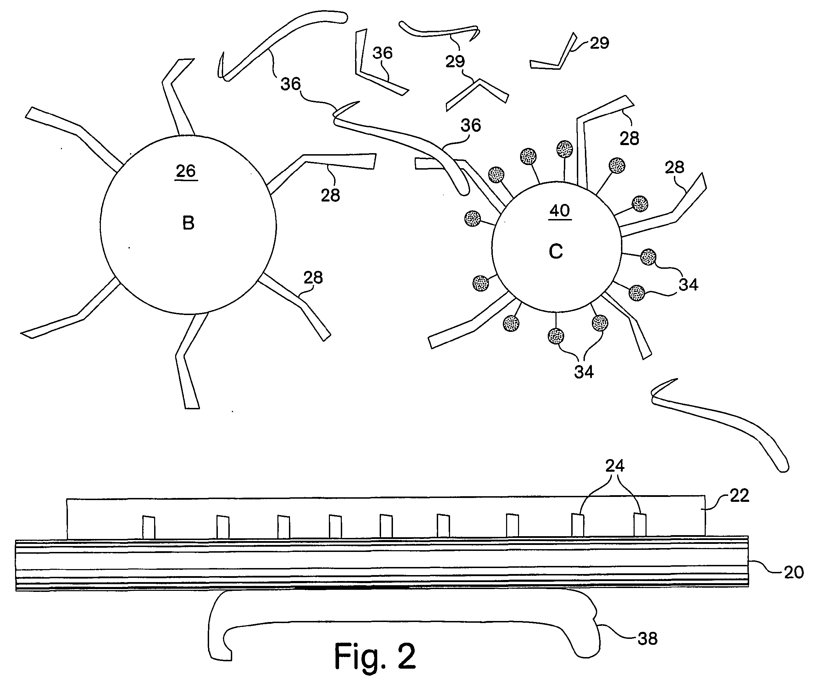

Referring now to Figure 2, another arrangement suitable for use as an assay as illustrated in Figure 1 is shown, but with modifications. In the arrangement of Figure 2, rather than a signaling entity 30 fastened to or adapted to be fastened to species 28, species 28 is immobilized relative to or adapted to be immobilized relative to individual signaling entities 34 by way of each of species 28 and signaling entities 34 being fastened to or adapted to be fastened to a colloid particle 40. Species 28 and signaling entities 34 can be adapted to be fastened to colloid particle 40 by any of a variety of chemical or biological techniques known in the art or described herein. It is particularly convenient, with the use of gold colloid particles 40, to attach signaling entities 34 and binding species 28 to a thiol which will bind to the surface of colloid 40. A SAM can be formed coating a surface of colloid 40 including the signaling entities 34 and binding species 28. In the arrangement of Figure 2, binding of binding species 28, fastened to each of bead 26 and colloid 40, to aggregate or fibril-forming species 36, followed by activation of magnet 38 to draw bead 26 to the proximity of electrode 20, in turn draws signaling entities 34 close to electrode 20 since signaling entities 34 are immobilized relative to bead 26. Electronic or electrochemical detection of signaling entities 34 near electrode 20 is routine using ACV or the like.

The assays illustrated in Figures 1 and 2 can be used for a variety of purposes. When used to determine whether aggregate-forming species 36 is present in a sample, a kit including bead 26, signaling entity 30 (Figure 1) or colloid particle 40 (Figure 2), and binding species 28 fastened to or adapted to be fastened to bead 26, entity 30, or colloid particle 40, is provided and mixed in solution with the sample. If aggregate-forming species 36 is present in the sample, then this is indicated by the proximity of signaling entity 34 near electrode 20 after mixture and activation of magnet 38. If aggregate-forming species 36 is not present, then signaling entity 34 should not be drawn to the electrode upon activation of the magnet.

In another technique, used for drug screening, aggregate-forming species 36 can be provided in known quantity in the sample in addition to a candidate drug for treatment of neurodegenerative disease, non-neurodegenerative disease, or other protein aggregation disease, or for treatment of a condition in which aggregation is desired. In such an arrangement, different candidate drugs will have different effects upon the binding of binding species 28 and in turn to signaling entity 30 or colloid particle 40 to bead 26 via the aggregate-forming species 36. Candidate drugs that inhibit this binding are candidates for treatment of neurodegenerative disease or other protein aggregation disease, or for treatment of a condition in which aggregation is desired. The assay can be established in such a way that a signal (proximity of signaling entity 34 to electrode 20) is proportional or otherwise related not only to the concentration of aggregate-forming species 36 in the sample, but to the degree of aggregation or fibril formation occurring within the sample. In such an arrangement, in a drug screening assay, the signal will be proportional or related to the degree to which a particular drug candidate prevents aggregation characteristic of a particular disease from aggregate or fibril-forming species 36 present in the sample, or the signal will be proportional or related to the degree to which a particular drug candidate enhances aggregation for physiological conditions where enhanced aggregation is desired.

In drug screening assays, it is often desirable to quantitatively determine the relationship between immobilization of bead 26 relative to signaling entities 34 in conjunction with concentration of aggregate-forming species 36 and/or concentration of drug candidate in a particular solution. In such an arrangement, it is desirable that binding species 28 bind aggregate-forming species 36 without conversion of binding species 28 to an aggregate-forming species, to maintain a 1:1 correlation between binding events and signal. This can be done by selecting binding species 28 and aggregate-forming species 36 from diverse biological species, e.g. from hamster and mouse, respectively, or a sequence derived from the same species that binds 1:1 with target.

In another arrangement, it is desirable to maximize sensitivity of the assay, rather than maintaining a 1:1 correlation between the binding events and signals or species in solution. In such an arrangement it is desirable to convert binding species 28 from species that simply will bind neurodegenerative aggregate-forming species 36, but are not aggregate-forming species themselves, into aggregate-forming species with the ability to convert other binding species to aggregate or fibril-forming species. In such an arrangement, with references to Figures 1 and 2, if only a very, very small amount of aggregate-forming species 36 is present in a sample (e.g. where as few as 100,000 molecules must be detected because that number of infectious prion molecules is sufficient to cause disease, characteristic of a sample from a patient in a very early stage neurodegenerative disease), "amplifier" species 29 can be provided in solution and mixed with a sample. In such an arrangement, even though a very small amount of aggregate-forming species 36 may be present in a sample, amplifier species 29 can be converted thereby to aggregate or fibril-forming species thereby rendering aggregate or fibril-forming species present in sufficient concentration to be detected using the assay. If no aggregate-forming species 36 is initially present in the sample, then amplifier species 29 will remain unconverted and little or no signal will be detected. Not only can amplifier species 29 amplify the concentration of aggregate-forming species 36 present in a sample to detectable levels, but it can facilitate massive aggregation of species 36 characteristic of neurodegenerative disease which in turn can form macro structures including many hundreds or thousands of beads, colloids, and/or signaling entities bound to many thousands of aggregate-forming species in a single macro structure. These structures can be easily detected electronically or electrochemically, and often visibly.

Amplifier species 29 can be selected among all of the species listed above for binding species 28, but need not be the same as binding species 28 in a particular assay. For example, binding species 28 in a particular assay can be a binding species, but without the ability to be converted to an aggregate-forming species, while amplifier species 29 can be convertible to define an aggregate-forming species. The latter situation (binding species 28 is not convertible but amplifier species 29 is convertible to an aggregate-forming species) it is useful in preventing particle/particle aggregation in the absence of a positive result (in the absence of aggregate-forming species 36 in a sample), but amplification of species 36, when it is provided in a sample, occurs, sensitizing the assay.
In conjunction with the use of amplifier species 29 to cause massive aggregation of a sample that initially includes insufficient aggregate-forming species 36 to cause massive aggregation (yet levels of species 36 desirably detected), electronic signaling entities and magnetic beads as illustrated in Figures 1 and 2 need not necessarily be used. In such an arrangement, a kit can be provided including colloid particles 40, binding species 28 fastened to or adapted to be fastened to colloid particles 40, and amplifier binding species 28. Such a kit can be mixed with a sample suspected of containing, or known to contain (in the case of drug screening), aggregate or fibril-forming species 36. Even at very low levels, species 36 can convert amplifier binding species 29 to aggregate or fibril-forming species, causing massive aggregation and the formation of macro structures easily detectable, for example, visible by the unaided human eye, e.g. color change, or visible under a microscope, detectable via a light scattering, absorbance or the like. In this arrangement, colloid particles 40 need not necessarily carry any auxiliary signaling entities.

In the arrangement described above with reference to Figures 1 and 2, it is desirable that binding species 28 be selected, and provided in concentration on surfaces of beads 26 and colloid particles 40 (where colloid particles are used), such that in the absence of auxiliary, non-surface immobilized aggregate-forming species 36, particle aggregation on particle/particle exposure is hindered within a time frame allowing comparison of (1) aggregation in the absence of auxiliary aggregate-forming species with (2) aggregation in the presence of auxiliary aggregate or fibril-forming species. That is, if no aggregate or fibril-forming species 36 is initially present in a sample, then aggregation of colloid particles may occur, but only slowly or to a limited extent, whereas if species 36 initially is present, then aggregation will occur more rapidly and/or to a much greater extent. In such an assay a control can include no sample, and the rate and/or aggregation level of the sample can be compared to that of the control.

It is not intended that the methods and assays as described in the immediately preceding paragraphs discussing neurodegenerative disease be limited to only a particular aggregate associated with a particular neurodegenerative disease. Indeed, the present invention contemplates detecting fibrils associated with a host of such diseases (e.g. Alzheimer's, Parkinson's, Creutzfeldt Jakob, etc.).

Provided is a variety of assays and components for assays, for detection of disease or determination of effectiveness of drugs for treatment of such disease. The assays can involve electronic or electrochemical detection of an interaction of a sample or drug screen assay with a sensing electrode, or can involve aggregation, which can be detected by a variety of techniques, including but not limited to visual inspection, density scanning, light transmission, absorbance, color change and/or light scattering. In connection with aggregation, binding species that can bind aggregate-forming species or can incorporate into pre-existing aggregates or fibrils, are attached to colloids (*e.g.* gold colloids) or other particles (*e.g.* fluorescent beads). These particles, optionally along with free binding species (species that are not attached to colloids but are suspended or dissolved in a fluid in which the colloids are suspended) are mixed with solutions that contain aggregates (*e.g.* patient samples). As the particles (*e.g.* colloids) become incorporated into the fibril, they render the fibril optically detectable. To inhibit peptides immobilized on colloids from interacting with each other, repellant groups - such as charged moieties (COOH, DNA, charged peptides) - can be fastened to surfaces of the colloids. This causes the immobilized peptides to interact with the fibril rather than with each other and thus reduces the occurrence of false positives in a diagnostic assay.

Also, flexible groups, such as glycol units can be chemically inserted between the nitrilo tri-acetic (NTA) moiety and the thiol portion. The introduction of more degrees of freedom would slow the rate of interaction by increasing the entropic cost of the reaction.

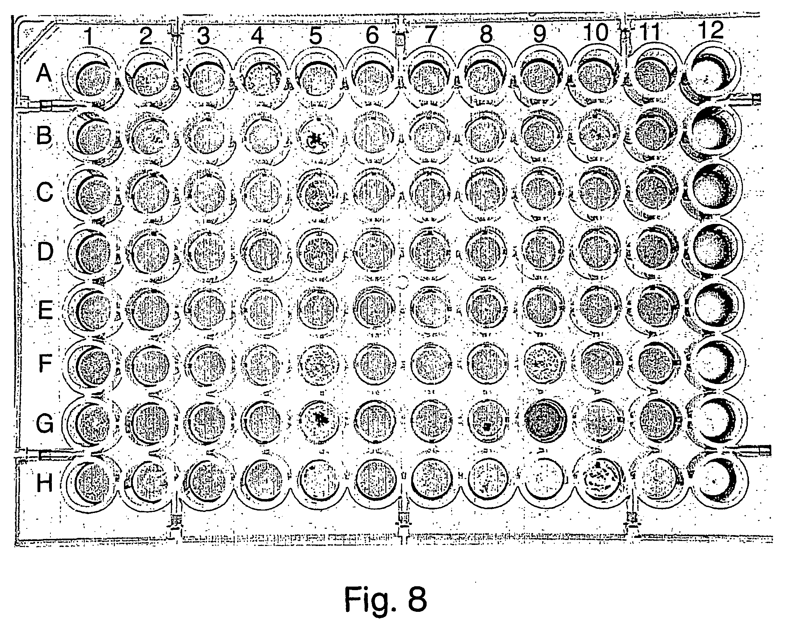

It is not intended that the present description be limited by the method of detection. To facilitate detection, the present description contemplates a variety of techniques. For example, a signaling entity, such as a dye, can be fastened to one end of a peptide capable of incorporating aggregates. As the signal-modified peptides incorporate into the fibril, they render it "visible" by concentrating the dye on its surface. Preferably, fluorescent moieties and quenching moieties are attached to the ends of separate peptides. At low concentration in solution, they do not interact frequently with each other; however, when co-localized on a fibril, a quenching reaction takes place, causing a change in the fluorescence of the solution. These changes can be analyzed using standard ELISA plate readers. Preferably, two compounds are attached to the termini of two separate peptides capable of incorporating into a fibril or plaque. The compounds are designed such that one compound modifies the second to render it active or detectable. The chemical modification is such that without the modification the compound does not fluoresce but after modification it does. Statistically, the reaction will happen orders of magnitude more frequently when the two compounds are co-localized on a fibril or plaque. Preferably, electronic or electrochemical interaction serves as a detection technique, for example, where the presence of a redox-active molecule near an electrode surface can be detected, indicating binding of a species immobilized relative to the redox-active molecule to a species immobilized relative to the electrode, or relative to a recruitable particle that can be drawn to the electrode, such as a magnetic bead. In another detection technique, an image of a sample can be digitized, then pattern recognition software is used to determine whether the sample contains aggregates. For example, a sample as shown in Figure 8 can be digitized using a CCD camera, and pattern recognition can be used to determine color, aggregate size, aggregate spatial distribution, relative amount of aggregation, etc. This allows automation of both diagnostic screening and drug screening. It is particularly useful, when doing screening of a large number of drugs, to be able to individually spatially address various samples. Techniques can be carried out using individually spatially-addressable regions, such as different wells of a multi-well plate (see Figure 8) and/or a plurality of electrodes. A plurality of electrodes can be arranged in individual wells of a multi-well plate, or the like.

It is particularly useful, when doing screening of a large number of drugs, to be able to individually spatially address various samples. Techniques can be carried out using individually spatially-addressable regions, such as different wells of a multi-well plate (see Figure 8) and/or a plurality of electrodes. A plurality of electrodes can be arranged in individual wells of a multi-well plate, or the like.

Regardless, the assays can be readily adapted to screen drug libraries for compounds that inhibit aggregate formation. For a drug-screening assay, one can attach binding species to colloids (or other particle) and incubate with solutions containing aggregate-forming or fibril-forming species and a drug candidate. The solutions may or may not be agitated to accelerate the incorporation of peptide into the fibrils. As the binding species are incorporated into the aggregates or fibrils, or aggregate-forming or fibril-forming species that then form aggregates or fibrils, they bring the attached colloids close to each other, which causes the colloid solution to change color (e.g. from a pink suspension to a dark blue precipitate in a clear solution). This transition is clearly visible by eye. By absorption spectrophotometry, the peak at 569 nm degrades as the colloids aggregate.

Preferably, the method employs peptides (that can form aggregates characteristic of neurodegenerative diseases or other protein aggregation diseases or other non-disease processes) which are modified with signaling and/or recruitment moieties and are incubated with a biologically-derived sample (or an in vitro mimic of such a sample) potentially containing converted or aggregated peptides indicative of the disease state. The existence of peptide plaques, aggregates, or fibrils is detected via the incorporation of the labeled peptides. This assay can be done in a variety of ways, as described below, in a system that allows fast and efficient detection of fibrillar or aggregated species.

For example, one element can be a magnetic particle that bears a peptide that participates in fibril or plaque formation. The second element can be in the form of a colloid derivatized with a heterologous SAM that presents both a peptide that participates in aggregate formation and a redox-active compound, such as a ferrocene derivative, which acts as a signaling moiety. Peptides that participate in fibril or plaque formation can catalyze the misfolding and spontaneous aggregation of other peptides, such as those attached to the beads and colloids. The presence of these fibrillar or converted species of peptide can be detected in a sample by detecting the association of peptides on magnetic beads with peptides on signaling colloids through their simultaneous incorporation into fibrils or aggregates.

Following an incubation period in which this aggregation is allowed to occur, an electromagnetic field can be applied that attracts the complex, which now contains magnetic particles as well as signaling colloids. In this system, magnetic particles provide a method of mixing biological recognition particles through a large sample volume and then rapidly recruiting the labeled targets to an electrode for detection. When an oscillating potential is applied to the electrode, a current peak at the oxidation potential that is characteristic of the redox-active metal indicates the presence of the aggregate at the electrode and, consequently, the presence of the aggregated peptides. In addition, free non-converted peptides can be added into the measurement solution to increase the rate of aggregation and/or the amount of material aggregated, thus acting as aggregation amplifiers. Aggregate formation can also be accelerated by varying the incubation and measurement conditions, including using frequency pulses and changes in temperature and electrical and magnetic fields. The magnetic particles can be drawn to a sensing electrode. Alternatively, where species are desirably recruited to an electrode or another location, gravity can be used. For example, components can be used that are not fluid self-suspendable, but will settle by gravity. Or, in other assays, fluid-suspendable components will aggregate to form non fluid-suspendable entities that will settle out of suspension.

The complexes can be detected using a variety of techniques including alternating current voltammetry (ACV). This detection method can be supplemented by the use of additional analysis techniques such as higher order harmonic analysis. These complexes may also be able to be detected by optical means such as surface plasmon resonance (SPR). In using the latter technique, large shifts in optical properties are caused by recruitment of particle/particles complexes to the detecting surface.

The aggregated state of these disease related peptides can also be detected by attaching potentially pathogenic peptides (binding species or aggregate or fibril-forming species) to either end of a linker, such that upon introduction of a polymerization seed or aggregate (aggregate or fibril-forming species optionally able to convert binding species to aggregate or fibril-forming species), the chains would form aggregates whose detectable properties would be altered from the original, non-aggregated state, e.g. by being visibly identifiable, rendered able to scatter light in light-scattering detection, change in viscosity in a flow detection system, electronic detection where an electronic signaling entity is linked to a component that forms part of the aggregate, etc. Alternatively, normal forms of pathogenic peptides can be attached to functionalized hydrogels or to ordered "holes" in gel-like materials. Since the introduction of a seed or protein aggregate would cause the conversion of the presented, soluble peptides into polymers or aggregates, that have altered optical properties, these aggregates could be optically detected.

Alternatively, the magnetic particle can be gold-coated and derivatized with a SAM that presents the desired peptide either by direct attachment of a peptide-thiol, or indirectly such as a DNA-peptide binding to a DNA-thiol incorporated in a SAM, or a histidine-tagged peptide binding to an NTA-thiol incorporated into a SAM. The colloid described herein may be a cluster of metal atoms, the preferred metal being gold. Because gold clusters can be made from as few as 20 gold atoms, colloids would be among the smallest possible particles with a diameter of only a few nanometers which could have significant advantages when incorporating these relatively small-sized fibril-forming peptides and metal-containing compounds into fibrils and plaques and maintaining them in suspension.

There can also be a linker inserted between the peptide and the particle or colloid or the recognition group that links the peptide to the particle or colloid so that steric hindrance does not interfere with the interaction between, e.g., a binding species and a fibril or aggregate-forming species. This linker or spacer can be made of a variety of materials, including glycols or amino acids. The length of the spacer can be varied to promote a shift in the oxidation potential of the electroactive signal detection substance by creating an increased hydrophobic environment due to the proximity of the aggregates.

The magnetic particle can also be replaced by a second colloid of larger dimensions and these larger colloids can be electromagnetically attracted to the electrode surface. However, they will only signal if decorated with satellite colloids that present signaling moieties. Alternatively, either colloid can be decorated with charged moieties that alter its mobility when an electromagnetic force is applied. All of the colloids can also be of similar size and an electromagnetic force can then be used to separate aggregated colloids from individual particles. In another variation, the first particle could be a heavier particle or colloid that can be mechanically mixed during incubation and subsequently allowed to accumulate on the electrode surface by sedimentation when measurements are taken.

These techniques can also be employed in a one-particle system. For example, magnetic beads can present pathogenic peptides and a transition metal complex. When pathogenic peptides in solution interact with peptides on beads to form aggregates, the local environment of the transition metal changes such that it will oxidize at a higher potential. The presence of pathogenic aggregates or fibrils are detected as a shift in the oxidation potential of the transition metal or as the appearance of a second oxidation peak at a lower potential, when the system is electrochemically analyzed. An alternative one-particle system includes magnetic particles that display the normal peptide and amplifying peptides in solution that carry transition metal complexes. These transition metals can be linked to dendrimers or polymers, either conducting or non-conducting. As pathogenic peptides in the test sample promote aggregate formation, the metal-containing compounds become intertwined with peptides attached to magnetic particles resulting in an electroactive and magnetic complex that can be recruited and electronically or electrochemically detected.

This assay system can also be constructed without using beads. Peptides with metal-containing compounds or peptide-thiols and transition metal complex thiols can be immobilized on or incorporated into SAMs on electrodes. Amplifying peptides may or may not be added to the measurement solution. If the test sample contains pathogenic or infectious peptides, then they attach to the surface immobilized peptides. This changes the local environment of the metal complexes and thus changes their oxidation potential. Amplifying peptides in the measurement solution may carry metal complexes that may be linked to dendrimers or polymers. If the test sample contains pathogenic peptides, then the aggregated species act to bridge the metalated peptides and the surface immobilized peptides. This interaction places the signaling moiety near the electrode surface and an electronic signal is transmitted to the electrode. Similarly, peptides incorporated into SAMs, formed on electrodes by either direct thiol attachment or by binding a His-tagged peptide to a pre-formed NTA-SAM, can be introduced to a sample solution that contains pathogenic peptides and also colloids that present both peptide and signaling moieties such as ferrocenes. Again, their integration into fibrils/aggregates brings the signaling moiety (in this case a colloid) close to the electrode and a signal is transmitted. Conversely, peptides derivatized with NTA groups can be attached to surfaces via an interaction with a peptide that contains a six histidine stretch. Amplifying peptides may or may not be added to the measurement solution.

These techniques can also be adapted to screening for potential drug candidates to treat these neurodegenerative diseases or other protein aggregation diseases or other non-disease processes. Specifically, in all of the described variations, a drug candidate can be added to the solution and its ability to inhibit, or enhance aggregate formation as a function of time and concentration, which would translate into dosage, measured. Various parameters, such as a loss of signal or a shift in the position of the current peak can be indicative of a positive, and therefore therapeutically useful, effect. In addition, the drug candidates can be attached to beads bearing redox-active, e.g. metal-containing, compounds, which can be colloids coated with SAMs either with or without NTA-thiols, that oxidize at a different potential than those metal-containing compounds attached to the colloids having peptides attached to them. One then looks for the different oxidation peak. The signaling metal-containing compound can also be removed from the colloid that has peptides attached and the single current peak from the drug-containing colloids measured. Also, a multiplexing drug screening device can be designed in which the drug candidates are added to microarrays and the whole array is flipped upside down onto a SAM-modified electrode lid for analysis.

This competitive inhibition assay could also be performed in a system in which the signal detection compound is on a colloid containing drug candidates. In this system, a magnetic bead without metal-containing signal detection compounds carries peptides. Free peptides in solution can interact with the peptides bound to these magnetic beads. Putative drug candidates can be then placed on colloids having metal-containing signal detection compounds. The metal-containing signal detection compounds on the colloids would signal if they interrupted the aggregation process and interacted directly with the peptide on the magnetic beads. One could also obtain a signal if the drug candidates interacted with aggregates. The latter information could be useful, but would likely require a subsequent multi-step assay to determine where the drug was acting.

Drug candidates are added to solutions containing pre-formed fibrils, peptides attached to electronic signaling colloids, and peptides attached to magnetic beads. Following an incubation period, magnetic beads are attracted to a sensing electrode and analyzed by ACV. A loss of signal indicates that the drug candidate has inhibited fibril formation.

The metastable protofibril can be selectively formed and purified to homogeneity by size exclusion chromatography, then used as the target fibril species for drug screening. Similarly, alpha-synnuclein, the protein that incorporates into fibrils characteristic of Parkinson's disease, can be His-tagged and attached to electronic signaling colloids and magnetic particles. These particles are incubated with synnuclein fibrils in solution or from a Parkinson's patient sample, then magnetically attracted to a sensing electrode for electronic analysis.

Pulsing the solutions with electromagnetic fields or mechanical agitation is used to accelerate the incorporation process.

As noted previously, it is also not intended that the assays of the present disclosure be limited to electronic or electrochemical interaction. In a particular embodiment, the method does not employ a sensing surface. Rather, the method involves aggregation, which can be detected by a variety of techniques, including but not limited to visual inspection, density scanning, light transmission, absorbance, color change and/or light scattering. Peptides, which can incorporate into pre-existing fibrils, are attached to colloids (*e.g.* gold colloids) or other particles (*e.g*. fluorescent beads). These peptide-presenting particles, along with free peptides, are mixed with solutions that contained fibrils (*e.g.* patient samples). As the particles (*e.g.* colloids) become incorporated into the fibril, they render the fibril optically detectable.

While it is not intended that the present description be limited to the particular details of how it is formatted into an assay, a convenient approach is the 96-well ELISA plate format. For the reaction, the following reagents can be added to the ELISA plate in phosphate buffer (10mM phosphate, pH 7.4, 100 mM NaCl) and the following concentrations can be conveniently used: a synthetic Aβ (1-40) peptide, with an N-terminal (His)₆ tag, at either 58.2 µM or 14µM, 30µL NTA/Ni⁺⁺-presenting gold colloids (preparation described below) and 0.6µM of Aβ-amyloid fibril "seeds". The ELISA plate can then be incubated at 37°C, without agitation, for a desired period (*e.g*. 30 minutes to 2 hour). At 1 hour, large dark red aggregate structures are clearly visible in the wells that contained 58.2 µM (His)₆-Aβ peptide. After a desired period (*e.g.* between 1 and 4.5 hours), the plate can be agitated briefly and then visually inspected. The aggregate structures are clearly visible in all wells that contained both (His)₆-Aβ peptide and Aβ fibril "seeds". These aggregate, fibrillar-like structures are also able to form in solutions that contained up to 50% fetal bovine serum (FBS), albeit at a slower rate. When the concentration of FBS is serially diluted and incubated with fibril seeds and (His)₆-Aβ peptide at constant concentration, the rate of visible fibril formation increases with decreasing FBS concentration, which indicates that the assay was specific for Aβ fibrils and not recognizing fibrillar species present in fetal serum. Wells that contained either fibrils or (His)₆-Aβ peptide, but not both, do not form visible aggregates. As a negative control, an irrelevant His-tagged peptide can be incubated with the colloids and seed fibrils. Such control solutions will not produce visible, fibrillar aggregates.

Using the above-described method, fibril seed concentrations as low as 10 picomolar are visibly distinguishable from solutions that contained colloids and Aβ peptide, but did not contain seed fibrils.

The colorimetric techniques described herein do not disrupt the assay process. A single fluid mixture is prepared and, without any transfer or other agitation required, the mixture can be observed to determine aggregate formation or lack thereof. Alternatively, a particular assay can be exposed to agitation or other form of energy. For example, it may be desirable in certain instances to determine whether introduction of energy into a particular system affects aggregate formation. Energy can be introduced in the form of agitation such as stirring, shaking, vibrating, sonicating, vortexing, or other mechanical agitation, exposure to electromagnetic radiation such as infrared, ultraviolet, or visible light, exposure to radio frequency energy, microwave radiation, or essentially any electromagnetic radiation at any portion of the spectrum, exposure to heat. In some instances, exposure of a system to energy will affect the rate of aggregation which can be indicative of the potential affect of the energy on neurodegenerative and other aberrant aggregation disease processes.

The colorametric techniques facilitate correlating drugs or candidate drugs with activity as a function of time or as a function of aggregate formation in connection with aggregate-forming biological processes. Preferably, this involves providing particles and binding species capable of binding aggregate-forming species fastened to or adapted to be fastened to the surface. The particles and binding species are exposed to a candidate drug suspected of affecting fibril formation. A first observable feature of the particles is determined indicative of effectiveness of a candidate drug in affecting aggregate formation at a first point in time. Then, at a second point in time, a second observable feature of the particles is determined indicative of effectiveness of a candidate drug in effecting aggregate formation. The assay can be constructed so that particles will bind to each other to varying degrees, or will become otherwise immobilized with respect to each other by being bound to a common surface. The candidate drug may be one that is suspected of inhibiting fibril formation, and the determining steps involve determining first and second observable features of the particles indicative of effectiveness of the candidate drug in inhibiting aggregate formation at the first and second points in time. Alternatively, the drug can be suspected of enhancing aggregate formation and its ability to do so can be determined at first and second points in time. The assay can involve exposure to auxiliary aggregate-forming species.

The observable features include directly visibly-identifiable changes (e.g., color change due to colloid aggregation or other direct visibly identifiable change described herein such as fluorescence quenching, etc.) The technique can be automated as well, involving spectroscopic determination of color change.

The first and second points in time, are relatively large. Specifically, they differ in time by at least 1 day or at least 1.5 days, or even at least 2 days. This can be useful in identifying drugs that may be particularly effective at late-stage aggregate associated physiological processes such as disease. Preferably, the first and second points in time differ by no more than 20 minutes, or less than 10 minutes, or less than 5 minutes or 1 minute, or even less than 30 seconds. This can be used to identify drugs that are particularly effective at very early-stage aggregate processes.

One advantage of this technique is that it can be conducted in a "one pot" assay. That is, the first and second observable features can be determined without disruption or agitation of the assay or exposure of external energy to the assay during and between the first and second determining steps. With this in mind, spectroscopic determination of an observable change preferably is done using visual spectroscopy. This is convenient because the assay looks for a change in the visible range (pink to blue), and visible spectroscopy does not add significant energy to the system. Even as visible spectroscopy is used, it is preferred that short spectra are taken at separate points in time, rather than exposing the assay to spectroscopic energy for long periods of time.

The observable features of the particles indicative of their binding status, and thereby the effectiveness of the candidate drug can be determined, if spectroscopically, at least two different wavelengths. For example, blue and pink each can be monitored to monitor a decrease in blue and an increase in pink.

Observable features also can include the formation of reticulum, i.e., visible observation of aggregate formation.

Preferably, the plurality of particles are exposed to the sample and an extent of aggregation of the particles is determined at least five hours after the exposing step. This can be done, also, at least 10 hours after the exposing step, at least 15 hours, or at least 20 hours after the exposing step. Preferably, no determination of aggregation occurs before these periods of time.

Preferably, the particles are exposed to the sample and aggregation is determined no later than one minute after the exposing step, or no later than 30 seconds, or no later than 10 seconds after the exposing step.

As noted, this can involve determination of drugs particularly useful for treatment of patients at different disease stages. This leads to one method involving administering, to a first patient exhibiting symptoms indicative of a first stage of a aggregate-associated process, a first drug for treatment of the process, wherein the first drug, upon exposure to an assay indicative of potential for effecting fibril formation, exhibits a first characteristic of affecting fibril formation. A second patient exhibiting symptoms indicative of a second stage of the process is administered a second drug, wherein the second drug, upon exposure to the assay, exhibits a second characteristic of affecting fibril formation. The aggregate-associated process can involve neurodegenerative disease, non-neurodegenerative disease, or non-disease processes. Additionally, changes in pH, salt and/or buffer concentration, etc. can be used to alter the rate of aggregate formation. This can be useful in involving correlation of aggregate size or formation progress, to effectiveness of certain drugs.

The description also involves using a cell that can produce an aggregate-forming species for a variety of purposes. In one technique, the cell can be exposed to a candidate drug for inhibition of aggregation-associated physiological processes such as neurodegenerative disease, and the potential of materials produced by the cell for formation of aggregates characteristic of neurodegenerative disease can be monitored. This can be useful for determining the effectiveness of the candidate drug for inhibition of the disease. Other techniques can be carried out as well. Use of a cell itself, rather than simply isolating aggregate-forming species produced by the cell and testing those species, allows one to carry out experiments and monitor drug efficacy in real time. This provides several advantages, including techniques that are simple, inexpensive, and rapid. One technique involves monitoring the production, e.g., secretion, of neurodegenerative disease aggregate or fibril-forming species from the cell as a function of time, for example over periods of time including 5 seconds, 30 seconds, and other small-time intervals including any number of minutes up to 1 day, and over longer periods of time including any number of days up to a week or even longer periods including 2 weeks, 3 weeks, etc. Monitoring production of these species by cells as a function of time allows determination of drug activity profiles as material is continually produced over time, i.e. ability of the drug to continually act in response to newly-produced material. Drug efficacy as a function of time also can be studied. This can allow determination of the effectiveness of the drug in acting in response to different materials produced by the cell at different points in time, for example points in time as described above. Also, stability (thermal) of the drug as a function of time can be determined, as can capacity of the drug to act upon increasing levels of materials secreted by the cell, etc.

Methods need not be limited to cells that have the capability to secrete aggregate-forming species. Alternatively, cells that produce or retain aggregate-forming species intracellularly, can be lysed prior to colloid addition. Although this is described for a neurodegenerative disease, it applied to non-neurodegenerative disease, and other non-disease processes; the cell may have the ability to produce or retain any aggregate-forming species or precursor of an aggregate-forming species.

In addition, assays can be constructed in different ways to derive information at different points in time relative to production of material in the cell. For example, a cell can be made to produce aggregate-forming species in the presence of a pre-mixed assay including colloids along with binding species fastened or fastenable to the colloids, or colloids and/or binding species can be added at any point in time in the process.

The technique also allows for the determination of the effectiveness of a candidate drug in affecting aggregate formation, where a specific mechanism associated with the formation may not be known. A candidate drug may be determined to be effective in affecting aggregate formation by affecting the production of the species by the cell, or affecting aggregate formation after aggregate forming species have been produced, or affecting of any stage of the process. Stages of the process can include transcription, translation, post-translational modification, and secretion.

The technique also allows for the monitoring, e.g. visualization, of the effectiveness of a candidate drug in affecting aggregate formation over time without intervention. In the assay described, no translation of any assay component, such as pipeting, agitation, or transfer of solution, is required. This can more effectively simulate the natural, biological environment, and thereby more effectively identify drugs that are effective in a natural environment. Any of the determination/visualization techniques described herein can be used, e.g. visualization with the unaided human eye of colloid/colloid aggregation, spectrophotometry, light-scattering, and other techniques described herein. Preferred techniques are visual inspection of a cell-containing medium to determine whether the medium remains pink in the presence of colloids, indicating no aggregate or fibril formation, or changes to blue, indicating colloid aggregation indicative of the presence of aggregate-forming species, or any color indication on the spectrum between pink and blue indicative of a corresponding intermediate state.

The technique also can involve visual or other detection of localization of aggregate or fibril formation, typically in concentrated areas surrounding cells, where a higher level of aggregate or fibril-forming species typically exists. Localization of aggregate or fibril-forming species can be seen as areas of increased peptide or colloid reticulum or as areas where the solution has cleared adjacent areas of increased peptide/colloid reticulum due to drawing of the aggregate or fibril-forming species into the areas of reticulum. The ability to observe aggregation around individual cells or clusters of cells allows for a more accurate assessment of the efficacy of a particular drug. Specifically, quantification of efficacy can be monitored more readily. For example, an assay can be conducted without knowledge of the number of cells involved, where a pattern of reticulum is easily recognizable and associated with a particular cell.

This technique also allows for determination of the toxicity of a candidate drug with respect to a cell.

Also provided is a series of assays and kits involving colloid-colloid interaction. Assays of first and second colloid particles that are allowed to become immobilized with respect to each other, or are prevented from becoming immobilized with respect to each other are described. Assays can indicate binding interactions, can involve enzymes that facilitate binding interactions, enzymes that cleave, drugs that inhibit binding interactions, or essentially any other binding interactions, the existence or lack thereof desirably being determined. Although this is described with respect to first and second colloid particles becoming immobilized with respect to each other, generally many colloid particles would be involved in a particular assay, and observed to determine whether aggregation of the colloid particles, characteristic of binding between them or binding to a common surface, occurs. Where aggregation does not occur a solution in which colloid particles are suspended remains pink. Where aggregation does occur, the solution will become blue or purple, and in many cases a visible reticulum (visible aggregation) will result. The reticulum can be determined visibly with the human eye, or microscopically.

One advantage is that color change, or lack thereof, can be determined spectroscopically. For example, a particular assay can be established for the determination of the ability of a candidate drug to inhibit binding between first and second species. The first and second species can be immobilized relative to (e.g., directly fastened to colloid particles), and the particles can be provided in separate containers (e.g., separate wells of a multi-well plate), and exposed to different candidate drugs. The wells can be measured spectroscopically for a change in absorption at a particular wavelength indicative of a color change resulting from colloid aggregation. Thus, one significant aspect involves automatically, via instrumentation, determining aggregation of colloid particles indicative of binding interaction or prevention thereof. In this and other assays of the disclosure species can first be immobilized relative to (e.g., fastened to) colloid particles and then exposed to a candidate drug, enzyme, or other species that may inhibit or facilitate binding, followed by exposure to the candidate drug or enzyme. Or first or second chemical or biological species can first be exposed to a candidate drug, enzyme, or the like followed by exposure to colloid particles to which the first and second chemical or biological species have the ability to fasten or become immobilized. Regardless of the order in which steps of assays are carried out, colloid-colloid aggregation is indicative of binding interactions or prevention thereof, and can be automated.

A variety of signaling entities can be immobilize relative to colloid particles, if desired. Signaling entities presented on colloids can be fluorescent molecules. Fluorescent-conjugated antibodies and other fluorescent fusion proteins, including green fluorescent proteins, are widely used in biomedical research and testing. These fluorescent proteins and molecules can easily be attached to gold colloids that also present putative binding partners either through affinity tags, EDC/NHS chemistry or by binding to a His-tagged protein A or G presented on NTA-SAM-coated colloids. Signaling entities such as fluorescent moieties also can be co-immobilized on a colloid via a biotin terminated ligand, or may be fastened via a chelate/metal/metal binding tag linkage. A fluorescent moiety may also be fastened by attaching it to an antibody and using a chelate/metal/metal binding tag with His-protein G to bind the antibody. The moieties can then be directly detected. Alternatively, one set of colloids bearing a first ligand bears a fluorescence moiety while a second set of colloids is derivatized to present a putative binding partner and a quenching moiety. If the ligand-receptor pair interact, then a measurable quenching of the fluorescence signal results. Drug candidates can be tested for their ability to disrupt these interactions.

First and second chemical or biological species can be immobilized relative to first and second colloid particles respectively, and other chemical or biological species can be immobilized with respect to other colloid particles in such studies. In some cases the first and second chemical or biological species will be identical, i.e., a plurality of colloid particles will carry the same immobilized entities. In other cases entities will differ. Chemical or biological species can be fastened directly to colloid particles, e.g., by being covalently attached to a SAM-forming species that fastens to the particles, or can be immobilized relative to colloid particles, e.g., by being fastened to a colloid particle, via another colloid particle. For example, a chemical species is immobilized with respect to a colloid particle if it is fastened to another colloid particle that is itself fastened to the colloid particle. Preferably, colloid aggregation is studied using chemical or biological species that are immobilized, or adapted to be immobilized relative to colloid particles by a binding interaction that does not involve a nucleic acid sequence binding to a complimentary nucleic acid sequence.

A variety of studies involving colloids/colloid aggregation can be carried out. One set of assays makes use of the effect of an absorptive or emissive species, immobilized with respect to a colloid particle, by a second species that is immobilized with respect to a second colloid particle, brought into proximity or removed from proximity of the first colloid particle by binding, cleavage, or other interaction desirably studied. For example, a fluorescent molecule may be immobilized with respect to a first colloid particle and a chemical species having the ability to quench fluorescence of the fluorescent molecule, i.e., effect emission of the fluorescent molecule, can be provided on a second colloid particle. Then, first and second species immobilized with respect to the first and second colloid particles, if they bind to each other, will bring the first and second colloid particles into proximity with each other, causing quenching of the fluorescent molecule. If the first and second species immobilize with respect to the first and second colloid particle, each can bind to a common analyte, then presence of the analyte will cause quenching of fluorescence, and absence of the analyte will avoid quenching.

A drug candidate may be studied for competition with the analyte for binding of one of the species, or binding with one site on the analyte. In this case, the analyte may be provided as a known species. Presence of the drug candidate will thus inhibit immobilization of the first and second colloid particles relative to each other, thus will inhibit quenching. Alternatively,enhancing emission or shifting the wavelength of emission or absorption of a first molecule, by a second molecule on a second colloid particle is described.

This colloid/colloid aggregation technique can be used to identify the binding partners of drugs or proteins of interest. This can be accomplished by attaching the drug or protein to one set of colloids and possible binding partners to other sets of colloids and assaying for a binding interaction between the two sets of colloids. Once a biological target of a drug or protein has been identified, candidate drugs can be added to the assay in the presence of the colloid-attached binding partners to disrupt binding of the drug or protein to the cognate ligand, allowing identification of synthetic mimics of the drug or protein on the first set of colloids. This technique is very useful in identifying the biological target of orphan drugs or uncharacterized proteins for diagnostic or drug-screening purposes. This technique will also allow identification of synthetic replacements or "mimics" of currently used drugs that are expensive or difficult to produce.

Preferably, an angiogenesis inhibitor is attached to one set of colloids (via an affinity tag linkage, chemical coupling, or nonspecific adsorption), and its biological target is attached to another set of colloids. For the unique case of an angiogenesis inhibitor that has two or more ligand-binding sites, such as endostatin, the ligand may be attached to one set of colloids and the angiogenesis inhibitor may be added in solution. Drug candidates are added and assayed for their ability to disrupt the binding interaction. Any drug that inhibits the interaction is then attached to a third set of colloids and assayed for binding to the angiogenesis inhibitor and the biological target of the angiogenesis inhibitor. A drug that binds to the biological target of the angiogenesis inhibitor and inhibits binding of the angiogenesis inhibitor to its target can be deemed a "mimic" of the angiogenesis inhibitor, and may be used as a replacement drug. This assay may be used to screen for mimics of virtually any drug. It is of specific interest for drug screening for synthetic replacements of angiogenesis inhibitors, which are both costly and difficult to produce. The assay can be used to identify synthetic replacements for endosatatin, through disruption of the endostatin-vitronectin or endostatin-RGD-prptide interactions, angiostatin, through disruption of the angiostatin-ATP-synthase or angiostatin-vitronectin interaction, or TNP-470 through disruption of the TNP-470-methionine-aminopeptidase interaction. As in other colloid/colloid assays, color change, fluorescence quenching, or other emissive molecule enhancement or suppression and the like can be indications of a result. Study of RGD/endostatin interaction is described in example 18 below.

This colloid/colloid aggregation technique also can be used for discovery of angiogenesis inhibitors or ligands involved in angiogenesis pathways. In one assay, suspected angiogenesis inhibitors or proteins involved in can be immobilized relative to (e.g., fastened to) a first colloid particle. Second colloid particles can be immobilized with respect to molecules that have been implicated in angiogenesis and/or metastasis, such as basement membrane proteins, integrins, or adhesion molecules. If a particular angiogenesis inhibitor binds to the basement membrane protein, integrin, or adhesion molecule immobilized on the second set of colloids, then the two sets of colloids will become immobilized with respect to each other and the binding interaction will become detectable by methods of the invention such as color change, precipitation, etc. Once an angiogenesis inhibitor is identified by this method, candidate drugs for disruption of the binding can be screened. If the drugs disrupt interactions, then colloid particles will not immobilize relative to each other or will do so to a lesser degree. This assay can be used with known angiogenesis inhibitors to identify or verify the biological targets of the angiogenesis inhibitors. Drug candidates can then be added to the assay to identify other drugs that act on the same biological target.

Another way in which colloid particles can be immobilized relative to each other in such assays involves colloid each being immobilized with respect to a common surface. The common surface can be a surface of another colloid particle presenting binding partners of species on the first colloid particles. The common surface can also be the surface of an article such as a membrane such as a nitrocellulose membrane, a chip surface, a surface of an article derivatized with an SAM, or the like. Preferably, the surface to which the colloid particles can bind includes binding sites at a high enough density so that if binding occurs (between species on the common surface and species on the colloid particles), the colloid particles will be brought into close enough proximity that detection (via color change characteristic of aggregation, quenching of fluorescence, or other property described herein) can occur.

Preferably, antibodies that recognize the HIV-related P24 protein can be fastened to or otherwise immobilized relative to colloid particles and exposed to a sample suspected of containing P24. If P24 is present, colloid particles will be brought into close proximity and detected as described herein. Also provided for is screening of candidate drugs for disruption of P24/antibody interaction.

There are many disease states in which a protein must be post-translationally modified by an enzyme before it becomes functional, and the present description provides techniques and components that can study such processes. Below are examples of enzyme modifications that promote a disease state. In these cases, it would be desirable to inhibit enzyme activity and, in so doing, inhibit the disease. However, there are cases in which one would want to accelerate enzyme activity as a therapeutic. Caspases are enzymes that are involved in promoting apoptosis, which is the term for "programmed cell death". Thus, it is advantageous to identify drugs, or biomolecules, that increase caspase activity and thus programmed cell death. Targeting these therapeutic agents to cancer cells enables the specific destruction of tumor cells.

Enzymes whose activity can be assayed using methods of the disclosure fall into two general categories: 1) enzymes that cleave a substrate; and 2) enzymes that add a piece onto a substrate.

An example of enzyme activity that falls into the first category, cleavage, is the processing of amyloid pre-cursor protein (APP). Cleavage products of APP form the aggregates and plaques associated Alzheimer's disease, thus enzymes that modify APP, such as presinnelin and beta- and gamma-secretase, are important targets of therapeutics. Cleavage generally produces peptides comprised of amino acids 1-40 or 1-42, with the 1-42 peptide being much more prone to aggregation and disease.

In other cases, it is advantageous to enhance enzyme cleavage activity. In such a case it is advantageous to screen for drugs or biomolecules that accelerate, either directly or indirectly, the cleavage of a certain substrate. For example, caspases are proteases that are critical players in the signaling cascade that triggers apoptosis or programmed cell death. Therefore, it is beneficial to identify agents that accelerate or trigger the activity of this class of enzyme. The drugs can then be targeted to tumor cells for their specific destruction with minimal damage to healthy cells.

Caspase 3 cleaves the sequence DXXD/X. It has been shown that caspase 3 cleaves protein kinase C (PKC) between the regulatory domain and the kinase domain. This allows the catalytic domain to function unchecked. This activity triggers apoptosis (programmed cell death). Mutants in the kinase domain of PKC did not trigger this type of (p53-dependent) apoptosis. Preferably, a peptide containing the Caspase 3 cleavage site is fastened to colloid particles. For example, the peptide can be synthesized or expressed with affinity tags at either end of the peptide, with gold colloids derivatized to present binding partners of the affinity tags. To this composition is added the enzyme that cleaves the substrate, either purified or as part of a mixture in the presence or absence of drug candidates, and their effect on cleavage is determined using techniques described herein with respect to colloid/colloid interaction. Drugs that stimulate cleavage, thus stimulate apoptosis, can be screened and identified, also using techniques described herein.

Another example of an enzyme that cleaves a substrate that could trigger apoptosis is now described. A complex comprised of cyclin dependent kinase 5¹⁰ (cdk5) and p35¹¹ are required for certain critical cellular processes in neurons. However, cleavage of p35 to p25 by the enzyme, calpain, creates the new protein complex p25/cdk5¹². This new complex is improperly localized and is constitutively active. The complex hyperphosphorylates tau which disrupts the cytoskeleton and triggers apoptosis in neurons. It has also been implied that cleavage of p35 to p25 can be enhanced by addition of beta-amyloid peptide 1-42.

It is clear that a drug able to inhibit cleavage of p35 would be useful as a therapeutic. For this reason it would be advantageous to identify agents that inhibit cleavage of p35 by calpain or other modifiers. The following is a prophetic example demonstrating how the present description can be used to facilitate high throughput screening of drugs to inhibit p35 proteolysis.

P35, which is the substrate for the enzyme calpain, is adapted (if necessary) to facilitate fastening, either directly or indirectly, to separate colloids that have also been derivatized to facilitate this fastening. For example, recombinant p35 protein could be expressed with a histidine tag at one terminus for attachment to a first gold colloid bearing NTA-Ni (which binds His-tags) and recombinantly fused to Glutathione-S-transferase (GST) at the other end for attachment to a second colloid bearing Glutathione. Alternatively, p35 could be spontaneously attached to a second colloid that has been derivatized to present an antibody against a portion of p35.

Initially, the modified p35 would not be mixed with the colloids. Rather, aliquots of p35 are separately incubated with calpain and a panel of drug candidates, suspected of having the ability to inhibit p35 cleavage by calpain. Following a suitable incubation period, two populations of gold colloids derivatized to simultaneously bind to two separate sites on the substrate, p35, are added. If when the gold colloids are added to the solutions, they remain the characteristic pink of the colloids free in suspension, then one can conclude that the substrate was cleaved and thus could not act to link two or more colloids into a colloid-peptide reticulum. If however, the solution turns from pink to blue, then one can infer that p35 was not cleaved, simultaneously linked 2 or more colloids together and that the drug candidate effectively inhibited calpain cleavage.

Alternatively, rather than monitor color change, one can observe the assay under a microscope to determine whether or not a peptide colloid reticulum had been formed. A drug that inhibited p35 cleavage would preserve the continuity of the peptide and leave both recognition sites connected to each other and to two separate colloids.

Preferably, beta-amyloid peptides (1-40 or 1-42, either soluble or in pre-formed aggregates) are added to the assay, along with the p35 substrate, purified calpain, a lysate containing calpain, or a lysate containing the cleavage activity and drug candidates suspected of inhibiting cleavage.

It is not intended that the description be limited by a specific enzyme or by using intact p35. Any portion of p35 that contains the cleavage site would be suitable for use as the assay substrate. Further, the substrate can be modified with any functionality that facilitates the simultaneous attachment of the substrate, directly or indirectly, to two colloids. The substrate can be modified with affinity tags, affinity sequences, biotin, and the like. It is preferred, but not required, that the substrate be modified with two different affinity tags to discourage binding of two sites on the peptide to two sites on the *same* colloid. Alternatively, the substrate can be unmodified but attached to two colloids via antibody recognition. Calpain can be added to the assay either in purified form or mixed with other enzymes, as in a cell lysate, and proteins, including beta-amyloid peptides.

The assay can readily be multiplexed by performing in 96-well plate format and/or by analyzing on an automated spectrophotometer.

Another example of an enzyme that can be studied in accordance with techniques described herein is matrix metallo proteins. Matrix metallo proteins are expressed on the surfaces of cancer cells, and are involved in cleavage of membrane proteins which is implicated in the progression of cancer and metastasis. In one technique, a substrate that can be cleaved by a matrix metallo protein (such substrates are known in the art) is modified for immobilization relative to two colloid particles. Exposure of this arrangement to the matrix metallo protein, in the presence of a candidate drug for inhibition of cleavage activity of the protein, indicates effectiveness of the candidate drug. Where the candidate drug effectively inhibits matrix metallo protein activity, the arrangement will be blue (aggregated colloids), and where the candidate drug does not effectively inhibit matrix metallo protein activity, the arrangement will be pink (dispersed colloids).

An example of enzyme activity that falls into the second category, addition, is the modification of the cancer-associated protein, Ras. Farnesyl protein transferase is an enzyme that adds a lipid-like group to Ras. This modification allows Ras to embed in the membrane and become functional. In this particular case, it becomes oncogenic. the substrate as with FPT, affinity tags or binding agents are added to each separate part of the substrate, such that a substrate bearing two tags will only result from the enzyme performing its function. In the specific case of the protein Ras, it can be recombinantly prepared with an affinity tag such as (His)₆ at the N-terminus to facilitate the later attachment of one colloid to the protein. The lipid-like group that the enzyme adds is a farnesyl pyrophosphate, which is easily synthesized in the lab with an affinity tag like biotin attached distal to the site of attachment to Ras. The affinity tagged Ras and the affinity tagged addition group are mixed with the enzyme and a drug candidate. After allowing suitable time for the reaction to proceed, colloids bearing binding partners for each affinity ligand are added to the test solution. Again an inhibition of the pink to blue color change indicates that the drug candidate inhibited the enzyme activity.

Determining colloid/colloid interactions, indicative of binding interactions between species immobilized with respect to colloids is described. The ability to form SAMs on colloids is one technique for linking species to colloids for such studies. Colloids can be linked to species desirably studied for their ability to bind to each other, such as biologically-relevant binding partners such as ligands and receptors, or can carry linked species that may have the ability to bind to a common entity, or which can each link to a species immobilized with respect to another colloid particle. This finds use in drug studies as well. Species for study can be linked to colloids by any technique described herein, e.g. metal binding tag/metal/chelate linkages. For example, metal binding tags such as histidine-tags can be attached to ligands and tagged putative binding partners can be incubated together with NTA/Ni(II) presenting colloids. A visible reticulum (aggregation visible by the human eye, by microscopy, etc.) will result if the two components are binding partners. Alternatively, the putative binding partners can be GST fusion proteins that would bind to glutathione presented on the colloid. Other linkers useful for attaching a binding species or other participant in assays of the invention to a surface include affinity tags. Affinity tags are well-known species used widely in biology, biochemistry, etc.

Preferably, compositions and methods can be used to detect target proteins and their interactions with other proteins, nucleic acids and small molecules. It is not meant that the invention be limited to studying interactions that involve proteins. The methods described herein can be applied to the detection of any two species interacting with each other. As described elsewhere, gold colloids have the intrinsic optical property that they appear pink when dispersed in a homogeneous solution. However, if the colloids are forced into close proximity to each other, then the solution turns toward the blue end of the spectrum. Proteins can be attached to gold colloids by a variety of methods described herein. The assay need not be limited to the detection of direct interactions. Ligands attached to colloids may cause the colloids to be drawn close together when the ligands recognize a common target, which may be a complex of biomolecules rather than a single target molecule.

Protein-colloid reticulums are also clearly visible in solutions that contain ligands and their binding partners, attached to colloids. Proteins or other molecules can be attached to colloids in a variety of ways including but not limited to attaching an affinity tag to the protein or molecule of interest and attaching a binding partner for the affinity tag to the colloid. Peptides terminated in with a sulfur can be directly attached to gold colloids or incorporated into self-assembled monolayers formed on colloids. Preferably, colloids are derivatized with self-assembled monolayers (SAMs) that present binding partners for convenient affinity tags that can be genetically fused to recombinant proteins of interest. For example, proteins can be expressed as fusion proteins such that they contain a label such as glutathione-S-transferase (GST). Glutathione, which is the binding partner of GST can be easily attached to a thiol and incorporated into a self-assembled monolayer on the surface of a gold colloid. Alternatively, unmodified proteins can be covalently attached to colloids bearing exposed carboxylates via EDC/NHS coupling chemistry.

Also anticipated is mixing a drug candidate with colloids presenting molecules that either directly or indirectly bind to each other and detecting a diminution of the color change from pink to blue or a reduction in the extent of visible reticulum formation. Conversely, methods can be used to identify molecules that facilitate the binding of two molecules to each other, either directly or indirectly.

The function and advantage of the above will be more fully understood from the examples below. The following examples are intended to illustrate the benefits of the present invention, but do not exemplify the full scope of the invention.

### Examples

The following examples serve to illustrate certain embodiments of the present disclosure and are not to be construed as limiting the scope thereof.
In the experimental disclosure which follows, the following abbreviations apply: eq (equivalents); µ (micron); M (Molar); µM (micromolar); mM (millimolar); N (Normal); mol (moles); mmol (millimoles); µmol (micromoles); nmol (nanomoles); g (grams); mg (milligrams); µg (micrograms); ng (nanograms); L (liters); ml (milliliters); µl (microliters); cm (centimeters); mm (millimeters); µm (micrometers); nm (nanometers); nM (nanomolar);°C (degrees Centigrade); PBS (phosphate buffered saline); U (units); d (days).

These examples describe techniques that are applicable to the investigation of aggregate formation of any type, whether it relates to neurodegenerative disease, non-neurodegenerative disease, or non-disease processes involving aggregation. With the benefit of the description herein, those of ordinary skill in the art can readily alter the examples below and apply them to any number of neurodegenerative or non-neurodegenerative diseases involving aggregation, and non-disease aggregation processes.

### Example 1: Spectrophotometic Detection of Fibrils

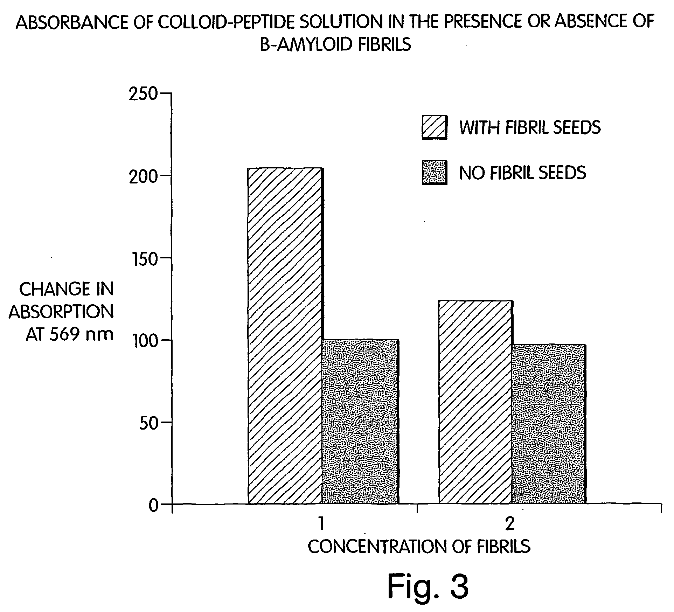

In this example, 100 µL aliquots that contained 14 µM His-Aβ (1-40) peptides and 30 µL of NTA-thiol colloids were incubated with either 1 µM of Aβ fibril seeds, 50 pM Aβ, fibril seeds or no fibrils. The mixtures were incubated at 37°C for 0.5 hours then transferred to a cuvette and placed in a Hitachi U-2000 Spectrophotometer. For each aliquot, the absorbance at 569 nm was recorded, then the cuvette was removed, rapped sharply against a hard surface to accelerate peptide incorporation into the fibril, allowed to rest for 10 minutes then re-scanned at 569 nm. A sharp decrease in absorption at 569 (as the fibrils precipitated out of solution) was observed for the solutions that contained fibril seeds, while those that contained only peptide remained stable (Figure 3). The height of the bars in the graph are the ratio of the initial measurement to the second measurement. 1) 1µM of Aβ fibril seeds (striped bar) and no fibrils present (solid bar); and 2) 50pM Aβ fibril seeds (striped bar) and no fibrils present (solid bar). The analysis process can be automated by using an absorption spectrophotometer that is compatible with a multi-well-plated format.

### Example 2: Colloid Preparation

In this example, 1.5 ml of commercially available gold colloid (Auro Dye by Amersham) were pelleted by centrifugation in a microfuge on high for 10 minutes. The pellet was resuspended in 100 µL of the storage buffer (sodium citrate and tween-20). 100 µL of a dimethyl formamide (DMF) solution containing 40 µM nitrilo tri-acetic acid (NTA)-thiol, 100 µM ferrocene-thiol, and 500 µM carboxy-terminated thiol was added (the ferrocene signaling entity is optional). Following a 3-hour incubation in the thiol solution, the colloids were pelleted and the supernatant discarded. They were then heat cycled in 100 µL of 400 µM tri-ethylene glycol-terminated thiol in DMF for 2 minutes at 55°C, 2 minutes at 37°C, 1 minute at 55°C, 2 minutes at 37°C, then room temperature for 10 minutes. Heat cycling results in the elimination of any species that are not in the lowest energy confirmation, resulting in a stable, close-packed, self-assembled monolayer. Heat cycling can be carried out with any of a wide variety of self-assembled monolayer-forming species. The colloids were then pelleted and 100 µL 100mM NaCl phosphate buffer were added. The colloids were then diluted 1:1 with 180 µM NiSO₄ in the colloid storage buffer.

### Example 3: Formation of a SAM-coded Electrode

In some of the embodiments described below, the examples involve SAM formation, collagen coating, cell growth, colloid formation, and Alternating Current Voltammetry (ACV). For SAM formation, glass microscope slides were sputtered with a layer of Ti followed by a layer of Au. Each electrode was incubated at RT for 0.5 hours with 300 µL of a DMF solution that contained 10% methyl-terminated thiol (HS-(CH2)15 CH3), 40% tri-ethylene glycol-terminated thiol, HS(CH₂)₁₁(CH₂CH2)₃OH, (formula) and 50% poly (ethynylphenyl) thiol (C₁₆H₁₀S). 2 ml of 400 µM tri-ethylene glycol-terminated thiol were then added to a scintillation vial containing the chip and the vial was heat cycled in a water bath as follows: 2 minutes @ 55°C; 2 minutes @ 37°C; 1 minute @ 55°C; 2 minutes @ 37°C then RT for 10 min. Electrodes were then dipped in EtOH, then sterile PBS to rinse.

### Example 4: Assay Demonstrating Extremely Sensitive Detection of Aggregates

Pre-formed aggregates, characteristic of neurodegenerative disease and other diseases characterized by aberrant protein aggregation, and other non-disease processes, can be sensitively detected by monitoring color change and visible fibril formation as a function of time.

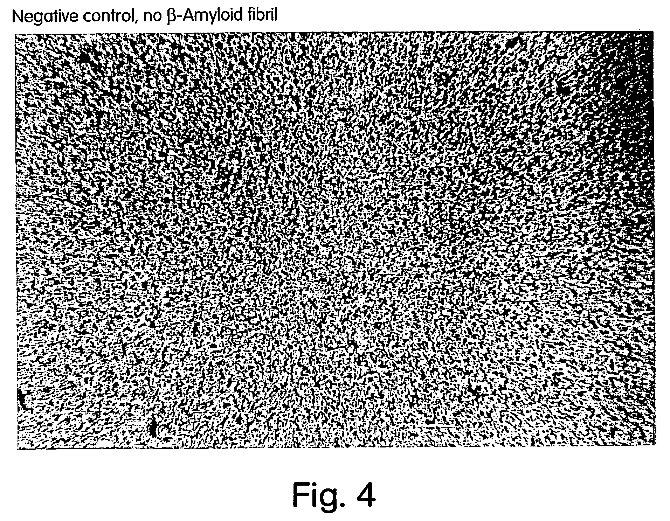

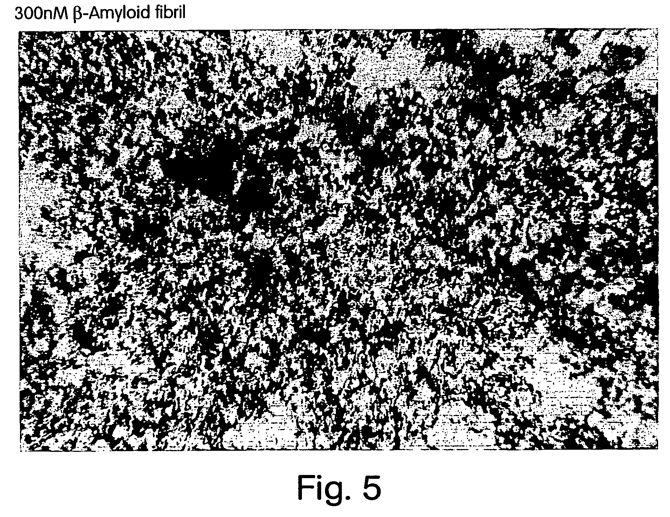

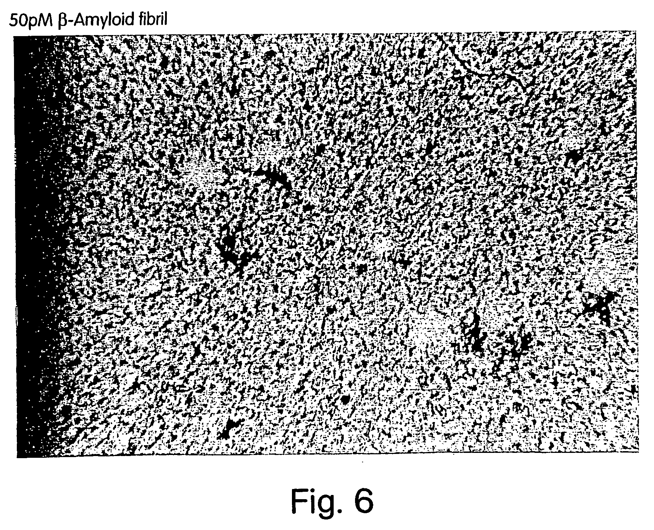

Gold colloids were derivatized with nitrilo tri-acetic acid/nickel self-assembled monolayers (NTA-Ni-SAMs), for the capture of histidine-tagged proteins, as described in Example 2. Colloids were coated with a low density of NTA-Ni (40µM NTA-thiol in a total thiol concentration of 1000:M) to inhibit the aggregation of nearest neighbor peptides, immobilized on a common colloidal particle. 30µL aliquots of the derivatized colloids were placed in wells of a 96-well plate. Histidine-tagged β-amyloid peptides (amino acids 1-40) were dissolved in phosphate buffer pH 7.4, then added to the colloid solutions such that the final concentration in a 100µL volume was 14µM. Pre-formed fibrils, made from synthetic non-histidine-tagged µ-amyloid peptides, were serially diluted and added to the solutions such that the final concentration of fibrils varied from 330 nanomolar to 50 picomolar. For every assay (each well), a negative control assay, in which no fibril was added, was performed (Figure 4). Solutions were incubated at 37° C for 1 hour and care was taken not to agitate the solutions. The color of the solutions that contained fibrils, changed from the characteristic pink color of gold colloids, to a deep purple/gray. Solutions that contained hisitidine-tagged µ-amyloid (1-40), but no pre-formed fibrils changed to a lighter purple color, and at a slower rate than solutions to which fibrils were added. Dark masses, in the centers of wells that contained pre-formed fibrils, were clearly visible with the unaided eye (Figure 5). Observation using 40-fold magnification with a dissecting microscope revealed large, purple reticulums. These aggregates were clearly suspended in the fluid medium rather than sedimented onto the bottom of the plate. Negative control wells showed a grainy precipitate, but no large aggregates. Visual examination allowed us to discriminate between wells that contained pre-formed fibril and those that did not, down to an added fibril concentration of 50 picomolar (Figure 6). Results were recorded by photographs taken with a Nikon camera (ASA 800 film) attached to an inverted microscope at 40-fold magnification.

### Example 5: Visualization Using Colloids

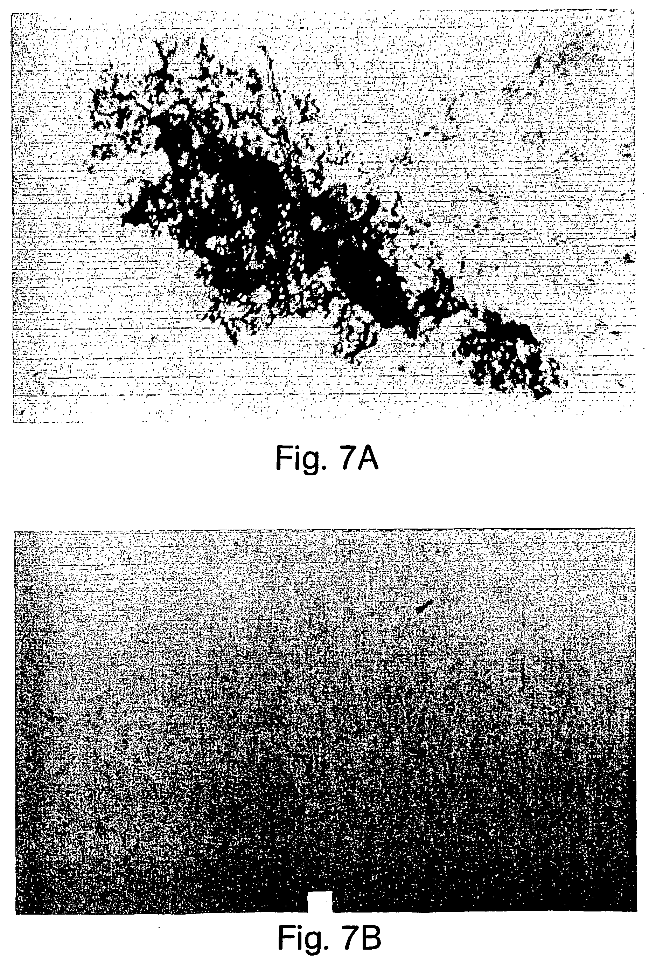

At higher peptide and fibril concentration, large colloid-decorated fibril structures are visible.
Colloids bearing 40µM NTA-Ni SAMs were prepared as described in Example 2. 30µL aliquots of colloids were added to wells of a 96-well plate. Histidine-tagged β-amyloid peptide (1-40) was added to achieve a final concentration of 20µM in a final volume of 100µL. Histidine-tagged β-amyloid peptide was added such that the final concentration was 20µM in a final volume of 100µM. Pre-formed β-amyloid fibrils were added such that the final concentration was 1µM. The sample was incubated at 37 degrees C for 20 minutes then rapped sharply three times to accelerate aggregation. After 1 hour continued incubation, at 37 degrees C, large colloid-fibril structures were clearly visible with the naked eye (Figure 7A). As colloids agglomerated onto the fibrillar structure, the surrounding solution cleared (no pink color). As a negative control, instead of a binding species fastened to the colloids, an irrelevant protein was fastened. The results are shown in Figure 7B.

### Example 6: Drug Screening

We determined conditions under which histidine-tagged β-amyloid (1-40) peptides would form aggregates. Drug candidates from the Sigma Aldrich RBI drug library were separately added to each assay solution to determine which drug candidates inhibited fibril formation characteristic of Alzheimer's disease. Colloids bearing NTA-Ni-SAMs (previously described) were added as a method to visualize results.

To each well of a 96-well plate were added: 30µL of NTA-SAM bearing colloids, 65µL of a 10.7µM solution of histidine-tagged β-amyloid (1-40) peptide (6.6µM final concentration), plus 5µL of a drug candidate such that the final concentration of each drug would be between 100-200µM. Plates containing solutions were incubated at 37 degrees C for several hours. During this time, plates were visually examined and photos were taken to record progress periodically.
Results:
Color change (from pink to blue) predicted which drugs inhibited fibril formation. At about 2 hours incubation time, one could clearly see with the unaided eye which wells contained drugs that had inhibited fibril formation by comparing the color of the solution to wells that contained positive control, i.e., no drug added and negative control, drug added but the histidine-tagged peptide was GST, not β-Amyloid.
Colloids agglomerated onto fibrils in the wells also enabling the identification of wells that contained drugs that inhibited fibril formation by noting the lack of visible aggregates in the wells. 40-fold magnification enhanced the ability to discriminate between the action of drugs in different wells.
Figure 8 is a photocopy of a photograph of a small molecule library, rack 1 after 72 hours incubating at 37 degrees C. The wells on the right-most side of the plate (column 12) are negative control wells that remained pink, in which His-tagged GST replaced the β-amyloid peptide. The next column to the left, column number 11 was the positive control that contained His-tagged β-amyloid and no drug candidate. Well G9 remained bright pink, indicating that the compound within that well inhibited fibril formation. Note wells A4, B5, C5, D5, F5, F9, G5, and H10 all displaying readily visibly-identifiable aggregate formation.
Monitoring color and or aggregate formation as a function of time under physiologic conditions are indicators of drug efficacy and drug stability.

### Example 7: Using peptides free in solution to amplify

Colloids presenting NTA-Ni-SAMs were prepared as described in example 2; 40µM NTA-thiol in a total thiol concentration of 1000µM was used. Histidine-tagged β-amyloid (1-40) peptides were added to achieve a final concentration of 58.2µM in 100µL final volume. As a negative control, an irrelevant histidine-tagged peptide, GST, was added to colloid solutions in place of the β-amyloid peptide. Solutions were incubated at 37 degrees C. Within 15 minutes large aggregates were clearly visible in solutions that contained histidine-tagged β-amyloid peptide. No structures were visible in control solutions. Solutions with β-amyloid peptide turned gray/purple while negative control solutions remained pink. These results are consistent with the idea that free β-amyloid peptides in solution became aggregated and acted to amplify or accelerate the aggregation process.

### Example 8: Electronic detection

Our strategy to electronically detect β-amyloid fibrils was to first incorporate histidine-tagged β-amyloid peptides (1-40) into pre-formed fibrils made from β-Amyloid 1-42 peptides. Colloids bearing NTA and a ferrocene derivative, for electronic signaling, were added so that at least some of the His-tagged β-amyloid peptides were attached to the colloidal particles. Our aim was to then add magnetic beads that bore binding ligands that would bind to the colloid-decorated fibrils and magnetically recruit them to the working electrode.

Colloids bearing NTA-SAMs were prepared as described in Example 2 (with the exception that 100µM octamethyl ferrocene-thiol was used in place of a standard ferrocene-thiol). 30µL of colloids were added to each assay solution. To facilitate alternating current voltammetry (ACV) analysis, without having to pipet solutions after peptide addition (which would accelerate fibril formation of peptides in the absence of pre-formed fibrils), assay solutions were mixed in a 1ml. capacity silicon gasket clamped over a gold-coated glass slide that had been derivatized with a self-assembled monolayer as described in Example 3, over a stationary magnet. Histidine-tagged β-amyloid peptide (amino acids 1-40) was added to a final concentration of 14µM in a 100µL volume. Pre-formed fibrils made up of β-amyloid peptide (1-42) were added to achieve a final concentration of 15µM. Negative control solutions were: (1) colloids, histidine-tagged GST plus fibrils, but no His-tagged β-amyloid peptide (1-40); (2) colloids, His-tagged β-amyloid peptide (1-40) but no fibrils; and (3) all the components expected to give a positive result, but measured at time zero, before His-tagged peptides could incorporate into the fibril. Solutions were incubated at 37 degrees C for 20 minutes. Commercially available magnetic beads (from Bang Labs and Prozyme) that were derivatized with Protein A (which binds the Fc portion of antibodies) were pre-bound to 1/10 their binding capacity with an antibody (purchased from Biosource Int.) that recognizes β-amyloid peptide 1-42, but not 1-40. 20µL of the antibody-presenting magnetic beads were injected into each solution immediately prior to ACV analysis (10 Hz.; 25mVolts overpotential; Ag/AgCl reference electrode with Pt auxiliary; SAM-coated gold chip acted as the working electrode).

### RESULTS:

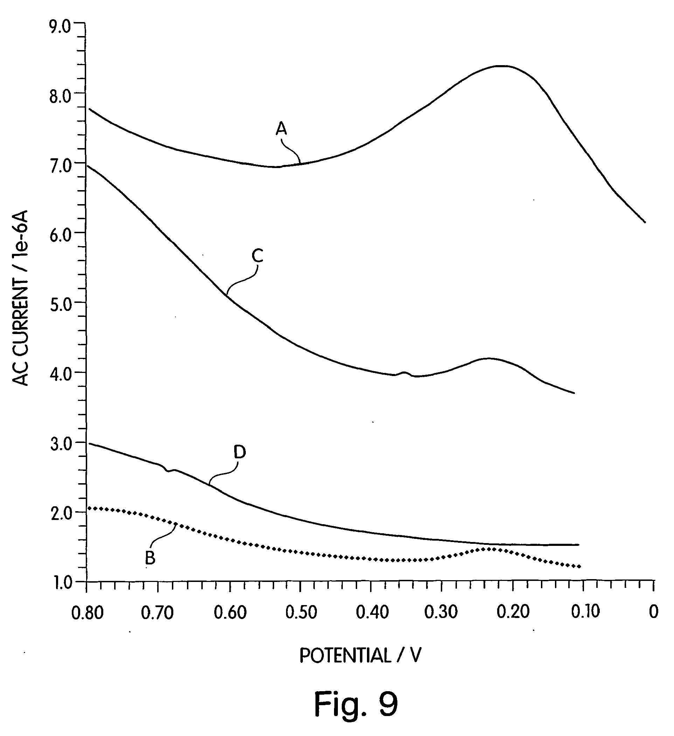

The solution that contained histidine-tagged β-amyloid peptides (1-40), colloids bearing NTA-Ni and ferrocene plus pre-formed fibrils, that had been incubated at 37 degrees C for 20 minutes, produced a current peak of 2.0 µAmps at the characteristic oxidation potential (220 mVolts) of the ferrocene derivative (octamethyl ferrocene) that was attached to the colloidal particle, see Figure 9, trace A.

An identical solution that was measured immediately, before histidine-tagged peptides could incorporate into the aggregate, produced an insignificant peak of approximately 0.17 µAmps at 220 mVolts, Figure 9, trace B.

Another negative control solution that contained all the components, but no pre-formed aggregate generated an insignificant peak of .022 µAmps (Figure 9, trace C).

A third negative control solution that contained pre-formed fibrils but in which histidine-tagged GST protein was attached to the colloids in place of histidine-tagged β-amyloid peptides produced no current peak (Figure 9, trace D).

### Example 9: Light Scattering Analysis

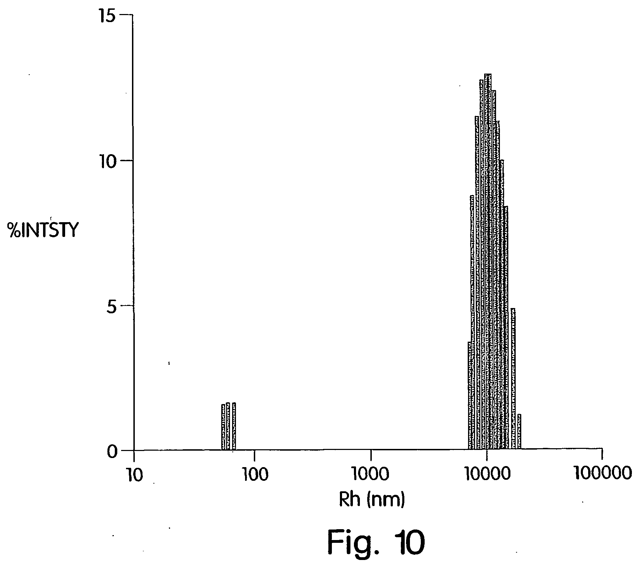

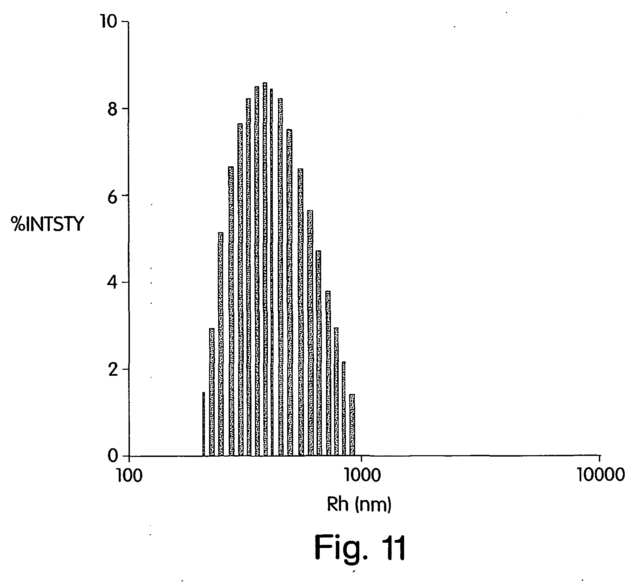

A commercially available light scattering device, that quantitates the average diameter of particles in solution, was used to analyze solutions that contained NTA-Ni-SAM coated colloids and histidine-tagged β-Amyloid (1-40) peptides in the presence or absence of pre-formed aggregate. Colloids were prepared as described in Example 2, histidine-tagged β-amyloid was added such that the final concentration was 10.2µM, in a final volume of 100µL. Pre-formed aggregates were added such that the final concentration was 2µM. Baseline measurements, of average particle diameter were taken of the reagents alone to ensure that none of the components were aggregating in the absence of β-amyloid fibrils.
Baseline Measurements:
1. 40µM NTA-SAM-derivatized colloids alone; diameter = 14.87 nm
2. colloids presenting histidine-tagged Aβ 1-40 peptides; diameter = 14.96 nm
3. colloids (without surface-immobilized His-tagged Aβ 1-40) mixed with 1µM β-amyloid peptide (1-42) pre-formed fibrils; diameter = 15.93 nm; t=0
Experiment:
1. colloids presenting histidine-tagged Aβ 1-40 peptides, mixed with 1µM β-amyloid peptide (1-42) pre-formed fibrils, measured at t=0 15.5 nm.
2. colloids presenting histidine-tagged Aβ 1-40 peptides, mixed with 1µM β-amyloid peptide (1-42) pre-formed fibrils, measured at t=1.5 hours, under high intensity lamp: diameter = greater than 10,000 nm, see Figure 10.
Negative controls:
3. colloids presenting histidine-tagged Aβ 1-40 peptides; buffer was added in place of the pre-formed fibrils; measured at t=0; diameter = 14.96 nm.
4. colloids presenting histidine-tagged Aβ 1-40 peptides; buffer was added in place of the pre-formed fibrils; measured at t=1.5; diameter = 579.7 nm, see Figure 11.

These results show that the amount of aggregate in a specimen can be quantitated, by measuring the diameter of resultant macro-structures. A light scattering device, that simultaneously measures the average particle diameter of solutions in 96-well plate format, is commercially available. Until now, attempts to use light scattering to distinguish between peptide monomers and β-amyloid aggregates have met with limited success because the diameters of both species are very small and at the lower limits of detection (β-amyloid fibrils are on the order of hundreds of nanometers), and the difference between monomers and aggregates is also small. However, the inventor illustrates that ligand-bearing colloids act to link together smaller, hard to detect fibrils, and stitch them into easily detectable macro-structures.

### Example 10: Specificity

To determine whether the β-amyloid peptides presented by the derivatized colloids would incorporate non-specifically into fibrillar species that would be present in a normal diagnostic sample, we performed the visual assay in fetal bovine serum (FBS). (FBS contains 100-fold more irrelevant protein than CSF, including other fibrillar species.) 1µM Aβ 1-42 fibrils were added to 30µL of 40µM NTA-SAM colloids that presented histidine-tagged Aβ 1-40 in solutions of varying FBS concentration. The aggregation of derivatized colloids onto fibrils did not increase with increasing FBS concentration, indicating that the assay was not prone to artifacts caused by other proteins or fibrillar species. Negative control solutions, which contained His-tagged Aβ 1-40 immobilized on colloids but did not contain pre-formed Aβ fibrils, remained pink; no structures were observed under 40- or 100-fold magnification.

### Example 11: Using the Extent of Visible Fibrillar Structure to Diagnose Alzheimer's Disease in CSF Samples

In a blinded study using CSF samples (unknown to the operator if one, both or neither came from AD patients), 30µM aliquots of colloids bearing 40µM NTA-SAMs, prepared as described in example 2, were added to wells of a 96-well plate. His-tagged Aβ (1-40) peptides were added to achieve a final concentration of 3.75µM. Aliquots of CSF from two Alzheimer's disease patients were added to the colloid/peptide solutions. The amount of CSF added was varied to give final CSF concentrations of either 12.5% or 25%. For each CSF sample tested, conditions were varied as follows: (1) sample solutions were doped or "seeded" with pre-formed Aβ fibrils (1 µM; 1-42); (2) solutions were seeded with pre-formed Aβ fibrils (0.1 µM; 1-42); (3) solutions were not seeded with anything; and (4) as a negative control no probe His-Aβ was immobilized on the colloids. After 3 hours at 37°C, fibrillar macro-structures were visible in all of the test solutions, except for negative controls, which do not include His-tagged Aβ and one sample from a patient (# 101) that did not contain any pre-formed fibrils. The extent of the fibrillar structures increased with increasing CSF concentration. Similarly, structure formation also increased with increasing seed concentration. The other CSF-containing solutions had turned from pink to purple but did not show any fibril aggregate formation under 40-fold magnification. Control solutions (no immobilized His-Aβ on colloids) remained pink and did not show any signs of structure formation. After overnight incubation (10 hours total), fibrils were also clearly visible in solutions that contained CSF from patient #109, although they were still much less extensive than those from the first patient. Like patient one, the extent of structure formation increased with increasing CSF concentration and with increasing β-amyloid seed concentration. Examination of sample records confirmed the CSF samples were both from Alzheimer's patients and that disease progression in the first patient (101), based on time since diagnosis and the Blessed Dementia Scale score, was about twice that of the second patient. The Blessed dementia scale score for patient 101 was 21 while the score for patient 109 was 12. β-amyloid concentration in patient 101 had been measured at 8 mgs/ml while # 109 was 4.6 mgs/ml.

### Example 12: Detection and Diagnosing of CJD

In this hypothetical example, colloids presenting NTA-Ni are added to a solution containing a His-tagged PrP protein in its soluble (unconverted form) such that at least some of the His-tagged peptides are attached to the colloids.

Diagnostic: Color Change: Normal Prion Protein (PrP), or a fragment such as amino acids 109-141 or 113-141, is derivitized to facilitate binding to SAM-coated colloids. For example, normal syrian hamster PrP is expressed with an affinity tag such as a 6-histidine tag. Histidine-tagged PrP is attached to NTA-SAM-derivitized colloids via NTA-Ni binding. A sample containing infectious PrP is added. Samples are incubated at 37°C to facilitate conversion of normal PrP to the protease-resistant form. Conversion of normal PrP to resistant form causes protein aggregation, and the colloids are forced close together causing a color change from pink to blue. Samples may be doped with normal unbound PrP proteins, or fragments thereof, to amplify the aggregation process, which is accelerated by protein concentration. Samples may also be subjected to mechanical agitation, light or sound energy to accelerate the aggregation process.

Drug Screen: Drug candidates are added to the PrP-bound colloids and infectious PrP sample and are allowed to bind. Samples are then incubated at 37°C to facilitate PrP conversion and aggregation. Samples containing drugs that inhibit binding of the peptide to the PrP or conversion of PrP to resistant form remain red, while samples containing drugs that are ineffective change color from red to blue.

Alternatively the drug candidate, PrP-bound colloid and infectious sample compositions are electronically or electrochemically analyzed after incubation. Samples containing drugs that inhibit binding of the peptide to the PrP or conversion of PrP to resistant form will not give an electronic signal or will produce an attenuated signal, while samples containing drugs that are ineffective will give an electronic signal due to precipitation of colloid aggregates onto the electrode.

A drug screening assay can also be devised that does not contain infectious material. A peptide consisting of amino acids 90-145 of the syrian hamster PrP sequence, previously shown to accelerate conversion of normal PrP to protease-resistant form, is added to the PrP-bound colloids. Samples are incubated at 37°C to facilitate conversion of normal PrP to resistant form. Conversion of normal PrP to resistant form causes protein aggregation, and the colloids are forced close together causing a color change from pink to blue. Alternatively the compositions can be electronically or electrochemically analyzed.

The invention itself can also be used to determine which PrP sequences are best for use as probe molecules attached to colloids and which sequences function as aggregation accelerators. Candidate *probe* sequence peptides are attached to colloids and tested alone to determine their intrinsic aggregation potential, which should be low (no color change) and then with infectious or converted PrP to assess their ability to bind the converted form of PrP (has color change). Peptide sequences that are candidates for aggregation accelerators are separately attached to colloids and tested alone and with converted PrP for their ability to aggregate and cause solution color change.
106-128 (KTNMKHMAGAAAAGAVVGGLGGY)
109-141 (MKHMAGAAAAGAVVGGLGGYMLGSAMSRPMMHF)
113-141 (AGAAAAGAWGGLGGYMLGSAMSRPMMHF)
119-141 (GAVVGGLGGYMLGSAMSRPMMHF)
117-141 (AAGAWGGLGGYMLGSAMSRPMMHF)
115-141 (AAAAGAWGGLGGYMLGSAMSRPMMHF)
113-141 (AGAAAAGAVVGGLGGYMLGSAMSRPMMHF)
Chabry J, Caughey B, Chesebro B, J Biol Chem 1998 May 22; 273(21): 13203-7; Residues 90-145 of Syrian hamster PrP, J. Mol. Biol. (1997) 270, 574-586.

### Example 13: Electronic Detection

In this hypothetical example, the presence of prion disease in a sample can also be electronically detected. Colloids presenting NTA-Ni and octamethyl ferrocene are added to a solution containing a His-tagged PrP protein in its soluble (unconverted form) such that at least some of the His-tagged peptides are attached to the colloids. Magnetic particles are also added to the assay that either present PrP or an antibody against it. A sample suspected of containing infectious PrP, is added. The infectious PrP will convert the normal PrP both on the colloid and free in solution, causing them to also participate in converting other proteins which results in massive aggregation. Massive aggregation results which incorporates both magnetic particles and colloidal particles into the same aggregates, which are then magnetically drawn to an electrode that can be SAM-coated and analyzed by ACV.

Diagnostic: Electronic. Infectious material can also be electronically detected. Normal PrP is bound to SAM-coated colloids as described previously that also bear a signaling moiety, such as a redox-active metal like ferrocene. Samples are then incubated at 37°C to facilitate PrP conversion and aggregation. Conversion of PrP to resistant form causes formation of insoluble aggregates, which precipitate onto the surface of an electrode, and deliver an electronic signal. Electrodes are analyzed using standard electrochemical analyzers and techniques such as alternating current voltammetry (ACV).

### Example 14: ELISA

In this prophetic example, a technique familiar to those skilled in molecular or cellular biology is used. Enzyme-linked Immunosorbent Assays (ELISA) (Current Protocols in Molecular Biology, Volume 2, Immunology 11.2, 1996, copyright from John Wiley and Sons Inc. 1994-1998). Normally when one performs an ELISA, a first species is directly or indirectly attached to a plastic substrate. A second species is added, the plate is washed and the presence of the second species is detected by binding to it a "secondary" antibody that also has a signaling capability; the secondary antibody is usually conjugated to an enzyme, typically horseradish peroxidase (HRPO), alkaline phosphatase (AP), or a fluorescent tag which is capable of performing a reaction on an added substrate that results in a color change (detected by a spectrophotometer), or a fluorescence labeling tag that can be detected by a fluorimeter (see, for e.g., "Localization of a passively transferred human recombinant monoclonal antibody to herpes simplex virus glycoprotein D to infected nerve fibers and sensory neurons in vivo", Sanna PP, Deerinck TJ, and Ellisman MH ,1999, Journal of Virology Oct. Vol 73 (10 8817-23)). Most often, for convenience, so that every antibody does not need to be conjugated to an enzyme, a mouse antibody is used as the specific recognition antibody, then an enzyme-conjugated rabbit-anti-mouse antibody is added.

Using the technology described herein, one can greatly increase the sensitivity of ELISAs and detect the presence of the immobilized target species using a natural ligand (protein or peptide) or a drug candidate as the probe molecule. The presence of the immobilized species is detected by binding to it a *ligand* attached to a colloid that also presents a plurality of horseradish peroxidase (HRPO) or alkaline phosphatase (AP). The enzyme can be conveniently linked to the colloid by a variety of means, including a histidine-tag attached directly to the enzyme, or by binding a mouse-anti-goat enzyme- conjugated antibody to a goat antibody that is attached to the colloid via a histidine-tagged protein G (Akerstom, B., Nielson, E., Bjorck, L. Jounal of Biological Chemistry, 1987 Oct. 5 Vol. 262 (28); pgs. 13388-91 and Fahnestock, S.R., Alexander, P., Nagle, J. and Filpula, D. (1986) Journal of Bacteriology Vol. 167, 870-880). By binding a ligand co-immobilized on a colloid with a plurality of enzymes, to the target species *in place of* a secondary antibody, the ratio of signaling molecules to binding events is increased by orders of magnitude. The binding of one antibody or ligand on the colloid to a presented antigen on an ELISA plate indirectly results in the binding of thousands or millions of enzymes. Alternatively, a known species can purposely be attached to wells of a 96-well plate so that one can probe with colloids that each present a separate drug candidate along with the signaling enzymes. Currently, it is not possible to do this with existing ELISA technology each drug candidate can not be conjugated to an enzyme. Alternatively, the natural ligand for the immobilized target can be presented on the colloid along with the signaling enzymes and drug candidates added to each well of the plate to disrupt the interaction. Unbound colloids and thus their signal are lost in a wash step. The target for the antibody- or ligand-presenting colloids can be a cell or a protein bound directly or indirectly (via another antibody or ligand) to an ELISA plate. The advantage to this modification of an ELISA is not only sensitivity, but also efficiency. Because several hundred signaling enzymes remain bound via the colloids to one antigen, substrate hydrolysis will occur more quickly, and less time will be needed for an adequate reading.

In this example, it is described how one would detect the presence of aggregate-forming or fibril-forming, protofibril, or aggregate species in a sample, or screening for drugs that would inhibit disease associated with aberrant protein aggregation, on an ELISA plate. A binding species that binds an aggregate or fibril-forming species, or an antibody to a fibril or aggregate-forming species, proto fibril or aggregate (specifically, an antibody to Aβ1-42) is immobilized in a well of an ELISA plate. In a diagnostic assay, the antibody then is exposed to a sample suspected of containing an aggregate-forming or fibril-forming species, and mixed with the sample is a binding species carrying a metal-binding tag (histidine-tag) optionally amplifier species, as described above, and colloid particles with a chelate coordinating a metal (NTA) and a non-electronic signaling entity each fastened to the particle. The non-electronic signaling entity could be a fluorescent tag, horse radish peroxidase, alkaline phosphatase, or the like.

Where one wants to screen for drugs using this assay, the same procedure is carried out but in the presence of a candidate drug for inhibiting aggregation processes.

### Example 15: Cell-Based Screening Assay for Candidate Drugs for Affecting Aggregate Formation at a Variety of Stages of Biochemical Progression

In this example, it is described how one tests a candidate drug for activity in affecting cellular processes involved in aggregation-associated disease including neurodegenerative disease and non- neurodegenerative disease, and non-disease processes. A whole cell assay was designed to enable the screening of drugs for their ability to affect, directly or indirectly, beta-amyloid production, processing including cleavage, and secretion (Wolfe, et al., Nature, 1999 April 8; 398 (6727):513-7; Haass, Nat. Med. December, 1999; 1 (12):1291-6; LaBlanc, et al., J Neurosci Res., April, 1992, 31(4):635-45).

Amyloid precursor protein (APP: the protein precursor of beta-amyloid) was stably transfected into human embryonic kidney cells. Selkoe, "The Cell Biology of Beta-Amyloid Precursor Protein and Presenilin in Alzheimer's Disease.", Trends in Cell Biology, 8:11, 447-453, November, 1998; Knops, et al. "Protein-type Specific-Inhibition of A-Beta Release By Bafilomycin A1, A Selective Inhibitor of Vaculor ATP ases", Journal of Biological Chemistry, 270:6, 2419-2422, February 10, 1995). Cells (100 ul vol at 40% confluency) were dispensed into a 96-well plate and grown in DMEM (Dulbecco modified eagle medium) in a CO₂ incubator at 37 degrees C for 21hrs. Colloids (30 ul) derivatized with NTA-SAMs (4% NTA in the thiol solution) were mixed with histidine-tagged beta-amyloid peptide (1-40) then added to each well of the micro titer plate. The final beta-amyloid concentration in each well was 17 uM. Control wells contained either: 1) histidine-tagged GST instead of beta-amyloid peptide; or 2) everything as above except no cells were present in the growth media; or 3) cells that were not transfected with APP.

When colloids and peptides were added at 21 hrs., only solutions containing cells expressing the APP plasmid, colloids and His-tagged beta-amyloid turned from the characteristic pink to purple. Solutions in the control wells remained pink.

Another batch of cells treated as described above, were allowed to grow for an additional 18 hrs. after colloids and beta-amyloid were added. These cells continued to extrude APP products (beta-amyloid 1-40 and 1-42). When observed under 40-fold magnification, a halo of accumulated colloid-peptide aggregates could be seen around each cell. Cells incubated with colloids and control his-tagged peptides showed no regional accumulation of colloids. Control cells (no APP transfection) incubated with beta-amyloid and colloids also showed no signs of colloid aggregation. Control solutions containing colloids, beta-amyloid and cell growth medium also did not show signs of colloid aggregation.

This example represents yet another aspect of the disclosure involving exposing a candidate drug for inhibition of neurodegenerative disease to a cell adapted to secrete neurodegenerative disease fibril- or aggregate-forming species, and determining the effect of the candidate drug on the aggregation potential of material secreted from the cell. This example also presents a technique for determining drug toxicity by determining the effect of the candidate drug on a cell.

### Example 16: Determination of Aggregation Characteristic of Tumor Suppression (p53)

In this hypothetical example, a strategy is described to screen for molecules that disrupt or enhance multimerization of p53. In some cases, the wild type protein is the target of the screen, while in other cases specific mutants will be targeted such that wild type function can be rescued. The multimerization domain of p53 (wild type or mutant) is expressed such that it is modified with a flexible linker that bears an affinity ligand to facilitate attachment to a gold colloid. In some cases, the tetramerization domain can be expressed without the nuclear export signal. The linker modification can be attached to either the N-terminus of the protein fragment (away from the multimerization domain) or at the C-terminus. Some compositions may contain a heterogeneous population such that the presence of the colloid will not sterically hinder multimerization. Also, unattached proteins may be added to the composition to avoid steric hindrance by the attached colloids.

The protein fragments are added to solutions containing gold colloids that have been modified to facilitate attachment of at least some of the proteins. If the protein fragments multimerize, then the solution will turn from its inherent pink color to a purple/blue. Drug candidates or biomolecules are added to some of the solutions. Again, a color change from pink to blue indicates multimerization. One can monitor a series of color changes. For example, protein fragments are added to the solution, multimerization occurs and the solution turns from pink to blue. Drug candidates or biomolecules are then added and a change from blue back to pink is detected which would indicate that a candidate molecule had disrupted multimerization.

Many of the mutations in p53 that occur in cancers involve the DNA binding domain of the protein. This disclosure can also be used to screen DNA sequences for their ability to bind to the mutants and also to identify molecules that restore binding to its native cognate DNA sequence. In this scheme, DNA (preferably ds but can also be single stranded) is attached to the colloids (via biotin-SA-SAMs or to commercially available streptavidin-coated colloids). p53 proteins or fragments thereof, that contain both the DNA binding domain and the tetramerization domain, (wt, muts, or both) are added to the solutions. Multimers of p53 that also bind to the colloid-immobilized DNA strands will induce condensation of the colloids and result in a color change from pink to blue. Drug candidates as well as biomolecules can be added to the compositions to test for their ability to promote DNA binding. In another embodiment, the proteins are added to separate batches of colloids that each bear a different DNA sequence. Since the proteins multimerize, a color change in the solution from pink to blue indicates that the protein complex bound to the DNA sequence presented on that particular batch of colloids. Alternatively, multimers of p53 that have mutations that affect DNA binding can be added along with drug candidates to solutions containing colloids presenting a DNA sequence (preferably the cognate sequence). This screen identifies molecules that alter DNA binding specificity.

### Example 17: Determination of Colloid/Colloid Linkage via Biotin/Streptavidin

A set of gold colloids was derivatized with self-assembled monolayers that incorporated biotin-thiols (SAM forming species included C11, 3 ethylene glycol units, then biotin) along with C16 carboxy-terminated thiols (a mixed SAM). Streptavidin, which has four binding sites for biotin, or a random protein (GST) was added to the colloid solutions. Solutions that contained streptavidin and its binding partner biotin, immediately turned blue while the control solutions did not.

### Example 18: Determination of Colloid/Colloid Linkage via Protein/Protein Recognition on NTA-presenting Colloids

600 microliters colloids, derivatized with a self-assembled monolayer that presents nitrilo tri-acetic acid, NTA (for the capture of histidine-tagged proteins), was mixed with 60 microliters of 500 micromolar histidine-tagged RGD-motif-containing peptide. 600 microliters of a second set of NTA-Ni presenting colloids was mixed with 60ul of a 500uM solution of GST, an irrelevant protein, as a negative control. Colloids were spun down and resuspended in phosphate buffer to remove residual unbound protein or peptide. Endostatin and Angiostatin, two proteins implicated in angiogenesis and suspected of binding to regions of vitronectin, such as the RGD peptide, were prepared by dialysis into phoshate-buffered saline solution and then 1:10 dilution into PBS from a stock concentration of 1mg/mL.

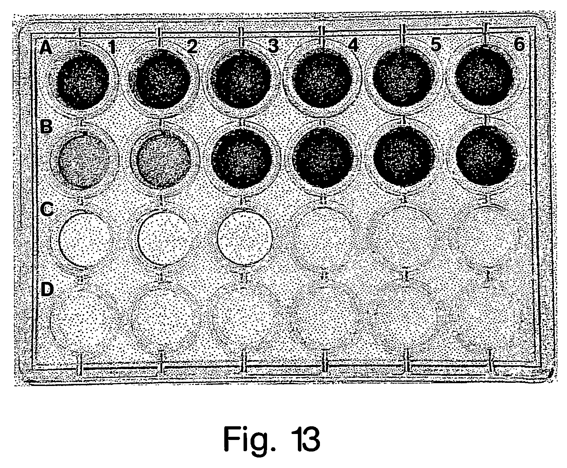

400 ul phosphate buffer (pH 7.4), 200ul either RGD-bound colloids or GST-bound colloids, and either 100ul endostatin or angiostatin, 50ul endostatin or angiostatin/ 50ul phosphate buffer, or 100ul phosphate buffer (as a negative control) were added to each well of a crystallization dish. Color change was monitored over time at room temperature. After approximately 15 minutes, a color change from red to blue was visible in wells that contained RGD- peptide-bound colloids and endostatin. The color change was more pronounced in the well that contained 100ul endostatin than in the well that contained 50ul endostatin/50ul phospate buffer. No color change occurred in the wells containing endostatin and GST-bound colloids or angiostatin and RGD- or GST-bound colloids. Figure 13 shows the result. Row A included colloids and angiostatin. Row B included colloids with endostatin. (Rows C and D are empty wells). Columns 1-3 have RGD peptide immobilized on the colloids. Rows 4-6 have an irrelevant protein, GST, immobilized on the colloid. Columns 1 and 4 contain 100 ml of the relevant drug (endostatin or angiostatin). The results show that endostatin binds to RGD-motif-containing peptides, and that endostatin is able to bind to two or more RGD-peptides, thus linking the colloids, and causing a color change from red to blue. The results were verified by gel electrophoresis. The RGD peptide and histidine-tagged GST were bound to a small amount of NTA-Ni agarose resin at saturating concentration. Endostatin was incubated with the resin and allowed to bind to the protein on the resin. The histidine-tagged proteins were then eluted using imidazole, and the samples were analyzed by SDS-PAGE. The results clearly showed that endostatin bound to the resin-immobilized RGD peptide and eluted with the protein off of the resin, while it did not bind to the resin-immobilized GST.

This assay could be easily adapted for screening of drug candidates that either mimic the RGD-binding characteristic of endostatin or bind to the RGD-binding domains on endostatin. Drug candidates could be added to the RGD-colloids in the presence of endostatin to look for drugs that inhibit the color change from red to blue.

### Example 19: Monitoring Drug Activity as a Function of Time for Drug Profiling

To determine at what stage of aggregate formation a particular drug is effective, for how long the drug is effective, and whether the drug is able to reserve aggregate formation, a drug profiling experiment was run in which many points were taken over time using a 96-well plate spectrophotometer.

To each well of a 96-well plate, 55 ul phosphate buffer (pH 7.4), 30 ul NTA-SAM derivitized colloids, 10 ul, 50 um His-AB, and 5ul drug candidate or DMSO (for control wells) were added. Negative control wells contained 10 ul, 50 um irrelevant Histidine-tagged peptide in place of His-AB, and DMSO in place of a drug candidate. Positive control wells did contain His-AB, but contained DMSO in place of a drug candidate. Absorbance at 530 nm was read every 5 minutes for 2 hours in a soft-max Pro 96-well spectrophotometer (molecular devices), while an identical 96-well plate was observed and photographed on the benchtop.

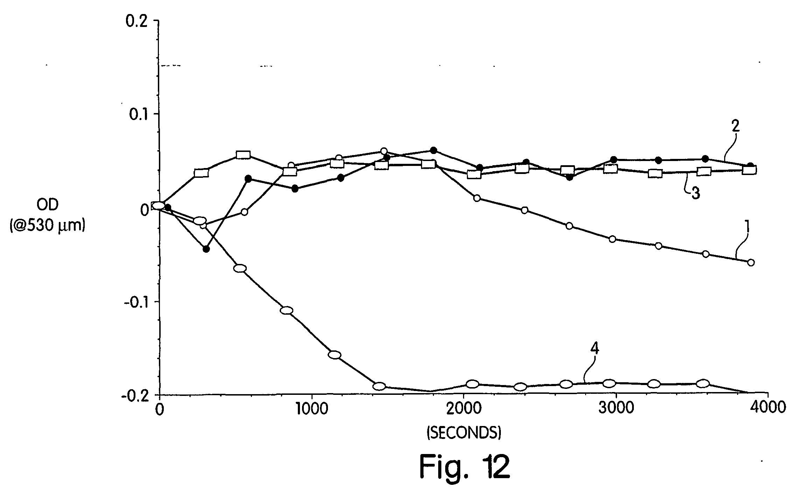

Negative control wells remained red, and positive control wells turned blue within 1 hour. Wells containing drug candidates varied in color over time depending on the effectiveness of the drug candidate. Absorbance at 530 nm was plotted against time for each well. Wells that turned blue, indicating no drug or no drug activity, showed a significant drop in absorption at 530 over time. Wells that contained drugs which inhibited aggregation showed a constant absorbance at 530 nm over the range of aggregate size on which the drug was effective, the drug was effective. In this manner, drug efficiency was determined. Figure 12 shows the drug profiles of two drugs that inhibited aggregation (1 and 2) plotted with a positive control (3) and a negative control (4). It can be seen that the two drugs work on different size aggregates for different periods of time.

The results of this experiment demonstrate the strength of another aspect of the disclosure, which involves determining an appropriate drug for treatment of a physiological process involving aggregation at a particular stage of the process or under conditions of particular aggregate size, or both. Referring to Figure 12, it can be seen that the drug represented by curve 1 shows greater effectiveness at an earlier stage of aggregate formation and/or with respect to smaller aggregates, and the drug represented by curve two shows greater effectiveness at a later stage of aggregate formation and/or with respect to larger aggregates. Conducting experiments such as this with a variety of candidate drugs can provide information for a treatment protocol based upon the stage of a physiological process and/or aggregate size in a patient.

### Example 20: Correlation of Color Change to the Extent of Aggregate Formation

This example demonstrates that colloid/colloid aggregation assays of the invention can be used to determine aggregate formation colorametrically and via formation of visible reticulum.

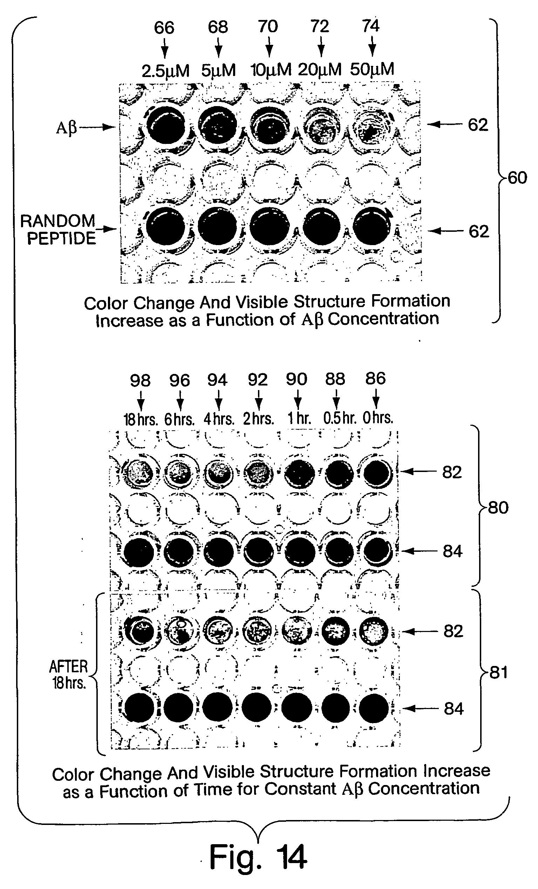

Mixtures were formed of wells of mutli-well plates including colloid particles, binding species capable of binding neurodegenerative aggregate-forming species adapted to be fastened to the colloid particles, and Aβ peptide. A control included a random peptide. Referring to Fig. 14, a first plate 60 includes rows 62 and 64 containing Aβ peptide and a random peptide respectively. Columns 66-74 contained 2.5 micromolar, 5 micromolar, 10, 20, and 50 micromolar Aβ peptide or random peptide, respectively. Aβ peptide was histidine-tagged, and colloids carried SAMs presenting chelate coordinating nickel. Row 64 contained an irrelevant histidine-tagged peptide. Each well contained one micromolar pre-formed aggregate.

As can be seen, row 62 containing binding species, colloid particles, and pre-formed aggregates turned from pink to blue, and especially at higher concentration, reticulum formation is clearly visible. A significant correlation between Aβ concentration and reticulum formation is observed.

Referring to plate 80, wells in row 82 contain 10 micromolar Aβ peptide (histidine-tagged), and NTA thiol-containing SAMs on colloids. Row 84 contained an irrelevant peptide at the same concentration, also histidine-tagged. All wells also contained one micromolar pre-formed aggregate. Components were introduced into the wells of the various rows 86-98 at different times, so that a single observation of the plate showed aggregate formation as a function of time. Row 86 was freshly prepared, row 88 had been prepared 0.5 hours prior to observation, and rows 90-98 had been prepared at 1, 2, 4, 6, and 18 hours prior to observation. Plate 81 is an observation of the identical plate as plate 80, after 18 hours. As can be seen, color change from pink to blue, and the formation of readily-visible reticulum, occurs as a function of time.

Those skilled in the art would readily appreciate that all parameters listed herein are meant to be exemplary and that actual parameters will depend upon the specific application for which the methods and apparatus of the present invention are used. It is, therefore, to be understood that the foregoing embodiments are presented by way of example only and that, within the scope of the appended claims, the invention may be practiced otherwise than as specifically described.
¹ MorelKopp MC, Kaplan C, proulle V, Jallu V, Melchior C, Peyruchaud O, Aurousseau MH, and Kieffer N, "A three amino acid deletion ", Blood, 90 (2) 669-677 1997.
² Zhang ZJ, Kundu GC, Yuan CJ, Ward JM, Lee EJ, DeMayo F, Westphal H, and Mukherjee AB, "Severe fibronectin-deposit renal glomerular disease in mice lacking uteroglobin", Science, 1997, 276 (5317) 1408-1412.
³ Li XF, Himanen JP, deLlano JJM, Padovan JC, Chait BT, and Manning JM, "Mutational analysis of sickle haemoglobin (Hb) gelation", Biotechnology and Applied Biochemistry, 1999, 29, 165-184.
⁴ Ref. Rhoades E., Agarwal J., Gafni A., "Aggregation of an amyloidogenic fragment of human islet amyloid polypeptide", Biochemica Et Biophysica Acta- Protein Structure and Molecular Enzymology, Vol. 1476 (2) 230-238, February, 2000.
⁵ Chene P., Bechter E., "P53 mutants without a functional tetramerization domain are not oncogenic", 1999, Journal of Molecular Biology, Vol. 286 (5) 1269-1274.
⁶ Chene P., Bechter E., "P53 mutants without a functional tetramerization domain are not oncogenic", 1999, Journal of Molecular Biology, Vol. 286 (5) 1269-1274.
⁷ Ryan KM, Ernst MK, Rice NR, and Vousden KH, Role of NF-kappa B in p53-mediated programmed cell death", Nature, 2000: 404 (6780) 892-897.
⁸ Stommel JM, Marchenko ND, Jimenez GS, Moll UM, Hope TJ and Wahl GM, "A leucine rich nuclear export signal in the p53 tetramerization domain: regulation of subcellular localization and p53 activity by NES masking", EMBO Journal, 1999, 18 (6) 1660-1672.
⁹ Nie Y, Li HH, Bula CM, and Liu XA, Stimulation of p53 binding by c-Abl requires the p53 c terminus and tetramerization", Molecular and Cellular Biology, 2000, 20 (3) 741-748.
¹⁰ Oshima, T. et al., "Targeted disruption of the cyclin-dependent kinase 5 gene results in abnormal corticogenesis, neuronal pathology and perinatal death", Proc. Natl. Acad. Sci. USA 93, 11173-11178 (1996).
¹¹ Chae, T. et al., "Mice lacking p35, a neuronal specific activator of Cdk5, display cortical lamination defects, seizures and adult lethality", Neuron 18, 29-42 (1997).
¹² Ming-Sum Lee, Young T. Kwon, Mingwei Li, Junmin Peng, Robert M. Friedlander and Li-Heui Tsai, "Neurotoxicity induces cleavage of p35 to p25 by calpain", 2000, Nature 405, pg. 360-364.

## Claims

1. A method comprising:
- providing:
a sample containing or suspected of containing non-neurodegenerative disease or non-disease aggregates, or non-neurodegenerative disease or non-disease aggregate-forming species;
gold colloid particles that bear a SAM on their surface, wherein the SAM is comprised of tri-ethylene glycol-terminated thiols, carboxylate-terminated thiols and thiols terminating in NTA-Ni groups; and
a plurality of histidine-tagged binding species specifically fastened or fastenable to the colloid particles, said binding species being capable of binding the aggregates or aggregate-forming species;
- exposing the sample to the binding species, allowing the binding species to interact with any of the aggregates or aggregate-forming species present in the sample; and
- determining the extent of aggregation of the aggregates or aggregate-forming species, by specifically fastening the colloid particles to the binding species, if not already fastened, and determining the extent of aggregation of the colloid particles.

2. A method as claimed in claim 1, wherein the colloid particles carry an auxiliary signalling entity.

3. A method as claimed in any of the preceding claims, wherein, the method comprises exposing the sample to the binding species and to the colloid particles, allowing the colloid particles to become fastened to the binding species, and determining the extent of aggregation of the colloid particles.

4. A method as claimed in any of the preceding claims, wherein the extent of aggregation of the colloid particles is determined by means of visualization by eye, under a microscope, by light scattering, by absorbance, or by colour changes.

5. A method as claimed in claim 4, wherein the extent of aggregation of the colloid particles is determined by means of any change in colour of the colloid solution upon exposure to the sample.

6. A method as claimed in any of the preceding claims, wherein the binding species is a peptide, protein or a fragment thereof.

7. A method as claimed in claim 6, wherein the binding species and the aggregate-forming species are homologous, preferably wherein the binding species and the aggregate-forming species are the same protein or protein fragments from diverse biological species.

8. A method as claimed in any of the preceding claims, wherein the sample contains or is suspected of containing non-neurodegenerative disease or non-disease aggregate-forming species.

9. A method as claimed in claim 8, comprising exposing the sample to the binding species in the presence of a candidate drug suspected of affecting aggregate formation.

10. A method as claimed in claim 8 or 9, wherein the binding species are not themselves aggregate-forming species, but said interaction converts the binding species to non-neurodegenerative disease or non-disease aggregate-forming species, whereupon the binding species participate in aggregation that is characteristic of the presence of the non-neurodegenerative disease or non-disease aggregate-forming species.

11. A method as claimed in claim 8 or 9, wherein said interaction is binding without conversion of the binding species to an aggregate-forming species;
further comprising allowing a second binding species, fastened to or adapted to be fastened to a surface of an article, to bind the aggregate-forming species.

12. A method as claimed in any of claims 1 to 7, wherein the sample contains or is suspected of containing non-neurodegenerative disease or non-disease aggregates.

13. A method as claimed in claim 12, comprising exposing the sample to the binding species in the presence of a candidate drug suspected of affecting aggregate formation.

14. A method as in claims 1 to 13, wherein the SAM completely covers the colloid surface such that non-specific adsorption is resisted and wherein no protein blocking step is carried out.

15. A method as in claims 1 to 13, wherein the SAM covered colloid is stable in a biologically relevant fluid with no detergents.

16. A method as in claims 1 to 13, wherein the SAM covered colloid is made comprising using tri-ethylene glycol-terminated thiols that are mixed into the thiol solution to prevent non-specific binding.

17. A method as in claims 1 to 13, wherein the SAM covered colloid is made comprising exposing colloids to self-assembling monolayer forming molecular species and a carboxylate and/or a surfactant.

18. A kit comprising:
gold colloid particles that bear a SAM on their surface, wherein the SAM is comprised of tri-ethylene glycol-terminated thiols, carboxylate-terminated thiols and thiols terminating in NTA-Ni groups; and
a plurality of histidine-tagged binding species specifically fastened or fastenable to the surface of the colloid particles, wherein the binding species are capable of binding non-neurodegenerative disease or non-disease aggregates, or non-neurodegenerative disease or non-disease aggregate-forming species.

19. A kit as claimed in claim 18, wherein the colloid particles are 10 - 30 nm in diameter.

20. A kit as claimed in any of claims 18 or 19, wherein the colloid particles carry an auxiliary signalling entity.

21. A kit as claimed in any of claims 18 to 20, wherein the binding species are peptides, proteins or fragments thereof that are homologous to the aggregate-forming species, preferably wherein the binding species and the aggregate-forming species are the same protein or protein fragments from diverse biological species.

22. A kit as claimed in any of claims 18 or 19, wherein the binding species are convertible to aggregate-forming species upon interaction with the aggregates or aggregate-forming species.

23. A kit as claimed in any of claims 18 or 19, comprising amplifier binding species that are capable of being converted to aggregate-forming species upon interaction with the aggregates or aggregate-forming species.

24. A kit as in claims 18 to 23, wherein the SAM completely covers the colloid surface such that non-specific adsorption is resisted and wherein no protein blocking step is carried out.

25. A kit as in claims 18 to 23, wherein the SAM covered colloid is stable in a biologically relevant fluid with no detergents.

26. A kit as in claims 18 to 23, wherein the SAM covered colloid is made comprising using tri-ethylene glycol-terminated thiols that are mixed into the thiol solution to prevent non-specific binding.

27. A kit as in claims 18 to 23, wherein the SAM covered colloid is made comprising exposing colloids to self-assembling monolayer forming molecular species and a carboxylate and/or a surfactant.

## Patentansprüche

1. Verfahren, das Folgendes beinhaltet:
- Bereitstellen:
einer Probe, die nicht mit einer neurodegenerativen Krankheit assoziierte oder nicht mit einer Krankheit assoziierte Aggregate oder nicht mit einer neurodegenerativen Krankheit oder nicht mit einer Krankheit assoziiertes Aggregat bildende Spezies enthält oder vermutlich enthält;
Goldkolloidpartikel, die eine SAM auf ihrer Oberfläche tragen, wobei die SAM aus Triethylenglykolterminierten Thiolen, Carboxylat-terminierten Thiolen und NTA-Ni-Gruppen-terminierten Thiolen zusammengesetzt ist; und
mehrere Histidin-markierte Bindungsspezies, die speziell an die Kolloidpartikel fixiert sind oder fixiert werden können, wobei die genannten Bindungsspezies die Aggregate oder Aggregat bildenden Spezies binden können;
- Aussetzen der Probe den Bindungsspezies, so dass die Bindungsspezies mit beliebigen der in der Probe vorhandenen Aggregate oder Aggregat bildenden Spezies interagieren können; und
- Ermitteln des Ausmaßes an Aggregation der Aggregate oder Aggregat bildenden Spezies durch spezielles Fixieren der Kolloidpartikel an die Bindungsspezies, wenn sie nicht bereits fixiert sind, und Ermitteln des Ausmaßes an Aggregation der Kolloidpartikel.

2. Verfahren nach Anspruch 1, wobei die Kolloidpartikel eine zusätzliche Signalentität tragen.

3. Verfahren nach einem der vorherigen Ansprüche, wobei das Verfahren das Aussetzen der Probe den Bindungsspezies und den Kolloidpartikeln, so dass die Kolloidpartikel an den Bindungsspezies fixiert werden können, und das Ermitteln des Ausmaßes an Aggregation der Kolloidpartikel beinhaltet.

4. Verfahren nach einem der vorherigen Ansprüche, wobei das Ausmaß an Aggregation der Kolloidpartikel durch Sehen mit dem Auge, unter einem Mikroskop, anhand von Lichtstreuung, anhand von Absorbanz oder anhand von Farbänderungen ermittelt wird.

5. Verfahren nach Anspruch 4, wobei das Ausmaß an Aggregation der Kolloidpartikel anhand einer beliebigen Farbänderung der Kolloidlösung nach Kontakt mit der Probe ermittelt wird.

6. Verfahren nach einem der vorherigen Ansprüche, wobei die Bindungsspezies ein Peptid, ein Protein oder ein Fragment davon ist.

7. Verfahren nach Anspruch 6, wobei die Bindungsspezies und die Aggregat bildende Spezies homolog sind, wobei die Bindungsspezies und die Aggregat bildende Spezies vorzugsweise dasselbe Protein oder dieselben Proteinfragmente von diversen biologischen Spezies sind.

8. Verfahren nach einem der vorherigen Ansprüche, wobei die Probe nicht mit einer neurodegenerativen Krankheit oder nicht mit einer Krankheit assoziiertes Aggregat bildende Spezies enthält oder vermutlich enthält.

9. Verfahren nach Anspruch 8, das das Aussetzen der Probe den Bindungsspezies in Anwesenheit eines vermutlich Aggregatbildung beeinflussenden Medikamentenkandidaten beinhaltet.

10. Verfahren nach Anspruch 8 oder 9, wobei die Bindungsspezies selbst keine Aggregat bildenden Spezies sind, aber die genannte Interaktion die Bindungsspezies in nicht mit einer neurodegenerativen Krankheit oder nicht mit einer Krankheit assoziiertes Aggregat bildende Spezies umwandelt, worauf sich die Bindungsspezies an der Aggregation beteiligen, die für die Anwesenheit der nicht mit einer neurodegenerativen Krankheit oder nicht mit einer Krankheit assoziiertes Aggregat bildenden Spezies charakteristisch sind.

11. Verfahren nach Anspruch 8 oder 9, wobei die genannte Interaktion Bindung ohne Umwandlung der Bindungsspezies in eine Aggregat bildende Spezies ist;
das ferner das Zulassen beinhaltet, dass eine zweite Bindungsspezies, die an einer Oberfläche eines Artikels fixiert oder für eine Fixierung daran ausgelegt ist, die aggregatbildende Spezies bindet.

12. Verfahren nach einem der Ansprüche 1 bis 7, wobei die Probe nicht mit einer neurodegenerativen Krankheit oder nicht mit einer Krankheit assoziierte Aggregate enthält oder vermutlich enthält.

13. Verfahren nach Anspruch 12, das das Aussetzen der Probe der Bindungsspezies in Anwesenheit eines vermutlich Aggregatbildung beeinflussenden Medikamentenkandidaten beinhaltet.

14. Verfahren nach den Ansprüchen 1 bis 13, wobei die SAM die Kolloidfläche so bedeckt, dass sie einer unspezifischen Adsorption widersteht, und wobei kein Proteinblockierungsschritt durchgeführt wird.

15. Verfahren nach den Ansprüchen 1 bis 13, wobei das mit einer SAM bedeckte Kolloid in einem biologisch relevanten Fluid ohne Detergenzien stabil ist.

16. Verfahren nach den Ansprüchen 1 bis 13, wobei das Verfahren zur Herstellung des mit einer SAM bedeckten Kolloids die Verwendung von Triethylenglykol-terminierten Thiolen beinhaltet, die zu der Thiollösung gemischt werden, um eine unspezifische Bindung zu verhindern.

17. Verfahren nach den Ansprüchen 1 bis 13, wobei das Verfahren zur Herstellung des mit einer SAM bedeckten Kolloids das Aussetzen von Kolloiden einer eine selbstaggregierende Monoschicht bildenden molekularen Spezies und einem Carboxylat und/oder einem Tensid beinhaltet.

18. Kit, der Folgendes umfasst:
Goldkolloidpartikel, die eine SAM auf ihrer Oberfläche tragen, wobei die SAM aus Triethylenglykol-terminierten Thiolen, Carboxylat-terminierten Thiolen und NTA-Ni-Gruppen-terminierten Thiolen besteht; und
mehrere Histidin-markierte Bindungsspezies, die speziell an der Oberfläche der Kolloidpartikel fixiert sind oder daran fixiert werden können, wobei die Bindungsspezies nicht mit einer neurodegenerativen Krankheit oder nicht mit einer Krankheit assoziierte Aggregate oder nicht mit einer neurodegenerativen Krankheit oder nicht mit einer Krankheit assoziiertes Aggregat bildende Spezies binden können.

19. Kit nach Anspruch 18, wobei die Kolloidpartikel einen Durchmesser von 10 - 30 nm haben.

20. Kit nach Anspruch 18 oder 19, wobei die Kolloidpartikel eine zusätzliche Signalentität tragen.

21. Kit nach einem der Ansprüche 18 bis 20, wobei die Bindungsspezies Peptide, Proteine oder Fragmente davon sind, die mit den aggregatbildenden Spezies homolog sind, wobei die Bindungsspezies und die aggregatbildenden Spezies vorzugsweise dasselbe Protein oder dieselben Proteinfragmente von diversen biologischen Spezies sind.

22. Kit nach Anspruch 18 oder 19, wobei die Bindungsspezies in aggregatbildenden Spezies nach Interaktion mit den Aggregaten oder aggregatbildenden Spezies umgewandelt werden können.

23. Kit nach Anspruch 18 oder 19, der Verstärkerbindungsspezies umfasst, die in aggregatbildende Spezies nach Interaktion mit den Aggregaten oder aggregatbildenden Spezies umgewandelt werden können.

24. Kit nach den Ansprüchen 18 bis 23, wobei die SAM die Kolloidoberfläche vollständig bedeckt, so dass sie einer unspezifischen Adsorption widersteht, und wobei kein Proteinblockierungsschritt durchgeführt wird.

25. Kit nach den Ansprüchen 18 bis 23, wobei das mit einer SAM bedeckte Kolloid in einem biologisch relevanten Fluid ohne Detergenzien stabil ist.

26. Kit nach den Ansprüchen 18 bis 23, wobei das Verfahren zur Herstellung des mit einer SAM bedeckten Kolloids die Verwendung von Triethylenglykol-terminierten Thiolen beinhaltet, die zu der Thiollösung gemischt werden, um unspezifische Bindung zu verhindern.

27. Kit nach den Ansprüchen 18 bis 23, wobei das Verfahren zur Herstellung des mit einer SAM bedeckten Kolloids das Aussetzen von Kolloiden einer eine selbstaggregierende Monoschicht bildenden molekularen Spezies und einem Carboxylat und/oder einem Tensid beinhaltet.

## Revendications

1. Procédé comprenant :
- la fourniture de :
un échantillon contenant ou supposé contenir des agrégats de maladie non neurodégénérative ou non pathologiques ou des espèces formant des agrégats de maladie non neurodégénérative ou non pathologiques ;
des particules colloïdales d'or qui portent une monocouche autoassemblée (SAM) sur leur surface, la SAM étant constituée de thiols à terminaison triéthylèneglycol, de thiols à terminaison carboxylate et de thiols se terminant en groupes NTA-Ni ; et
une pluralité d'espèces de liaison étiquetées par l'histidine fixées ou pouvant être fixées de façon spécifique aux particules colloïdales, lesdites espèces de liaison étant capables de se lier aux agrégats ou aux espèces formant des agrégats ;
- l'exposition de l'échantillon aux espèces de liaison, le fait de laisser les espèces de liaison interagir avec l'un quelconque des agrégats ou l'une quelconque des espèces formant des agrégats présents dans l'échantillon ; et
- la détermination du degré d'agrégation des agrégats ou des espèces formant des agrégats, par fixation de façon spécifique des particules colloïdales aux espèces de liaison, si elles ne sont pas déjà fixées, et détermination du degré d'agrégation des particules colloïdales.

2. Procédé selon la revendication 1, dans lequel les particules colloïdales portent une entité de signalisation auxiliaire.

3. Procédé selon l'une quelconque des revendications précédentes, le procédé comprenant l'exposition de l'échantillon aux espèces de liaison et aux particules colloïdales, le fait de laisser les particules colloïdales se fixer aux espèces de liaison et la détermination du degré d'agrégation des particules colloïdales.

4. Procédé selon l'une quelconque des revendications précédentes, dans lequel le degré d'agrégation des particules colloïdales est déterminé au moyen d'une visualisation à l'oeil, sous un microscope, par la diffusion de la lumière, par l'absorbance ou par des changements de couleur.

5. Procédé selon la revendication 4, dans lequel le degré d'agrégation des particules colloïdales est déterminé au moyen d'un quelconque changement de couleur de la solution colloïdale lors de l'exposition à l'échantillon.

6. Procédé selon l'une quelconque des revendications précédentes, dans lequel l'espèce de liaison est un peptide, une protéine ou un fragment de ceux-ci.

7. Procédé selon la revendication 6, dans lequel les espèces de liaison et les espèces formant des agrégats sont homologues, de préférence dans lequel les espèces de liaison et les espèces formant des agrégats sont la même protéine ou les mêmes fragments de protéine provenant de diverses espèces biologiques.

8. Procédé selon l'une quelconque des revendications précédentes, dans lequel l'échantillon contient ou est supposé contenir des espèces formant des agrégats de maladie non neurodégénérative ou non pathologiques.

9. Procédé selon la revendication 8, comprenant l'exposition de l'échantillon aux espèces de liaison en présence d'un médicament candidat supposé avoir une incidence sur la formation d'agrégats.

10. Procédé selon la revendication 8 ou 9, dans lequel les espèces de liaison ne sont pas elles-mêmes des espèces formant des agrégats, mais ladite interaction convertit les espèces de liaison en espèces formant des agrégats de maladie non neurodégénérative ou non pathologiques, sur quoi les espèces de liaison participent à l'agrégation qui est caractéristique de la présence d'espèces formant des agrégats de maladie non neurodégénérative ou non pathologiques.

11. Procédé selon la revendication 8 ou 9, dans lequel ladite interaction est une liaison sans conversion des espèces de liaison en une espèce formant des agrégats ;
comprenant en outre le fait de laisser une seconde espèce de liaison, fixée ou conçue pour être fixée à une surface d'un article, se lier aux espèces formant des agrégats.

12. Procédé selon l'une quelconque des revendications 1 à 7, dans lequel l'échantillon contient ou est supposé contenir des agrégats de maladie non neurodégénérative ou non pathologiques.

13. Procédé selon la revendication 12, comprenant l'exposition de l'échantillon aux espèces de liaison en présence d'un médicament candidat supposé avoir une incidence sur la formation d'agrégats.

14. Procédé selon les revendications 1 à 13, dans lequel la SAM recouvre complètement la surface du colloïde de façon telle qu'une adsorption non spécifique est entravée et dans lequel aucune étape de blocage de protéines n'est effectuée.

15. Procédé selon les revendications 1 à 13, dans lequel le colloïde recouvert de la SAM est stable dans un liquide biologiquement pertinent sans détergents.

16. Procédé selon les revendications 1 à 13, dans lequel le colloïde recouvert de la SAM est formé par le fait de comprendre l'utilisation de thiols à terminaison triéthylèneglycol qui sont mélangés dans la solution de thiols pour empêcher une liaison non spécifique.

17. Procédé selon les revendications 1 à 13, dans lequel le colloïde recouvert de la SAM est formé par le fait de comprendre l'exposition de colloïdes à des espèces moléculaires formant une monocouche autoassemblée et un carboxylate et/ou un tensioactif.

18. Kit comprenant :
des particules colloïdales d'or qui portent une SAM sur leur surface, la SAM étant constituée de thiols à terminaison triéthylèneglycol, de thiols à terminaison carboxylate et de thiols se terminant en groupes NTA-Ni ; et
une pluralité d'espèces de liaison étiquetées par l'histidine fixées ou pouvant être fixées de façon spécifique à la surface des particules colloïdales, les espèces de liaison étant capables de se lier à des agrégats de maladie non neurodégénérative ou non pathologiques ou à des espèces formant des agrégats de maladie non neurodégénérative ou non pathologiques.

19. Kit selon la revendication 18, dans lequel les particules colloïdales ont un diamètre de 10 à 30 nm.

20. Kit selon l'une quelconque des revendications 18 ou 19, dans lequel les particules colloïdales portent une entité de signalisation auxiliaire.

21. Kit selon l'une quelconque des revendications 18 à 20, dans lequel les espèces de liaison sont des peptides, des protéines ou des fragments de ceux-ci qui sont homologues aux espèces formant des agrégats, de préférence dans lequel les espèces de liaison et les espèces formant des agrégats sont la même protéine ou les mêmes fragments de protéine provenant de diverses espèces biologiques.

22. Kit selon l'une quelconque des revendications 18 ou 19, dans lequel les espèces de liaison sont convertibles en espèces formant des agrégats lors de l'interaction avec les agrégats ou les espèces formant des agrégats.

23. Kit selon l'une quelconque des revendications 18 ou 19, comprenant des espèces de liaison amplificatrices qui sont capables d'être converties en espèces formant des agrégats lors de l'interaction avec les agrégats ou les espèces formant des agrégats.

24. Kit selon les revendications 18 à 23, dans lequel la SAM recouvre complètement la surface du colloïde de façon telle qu'une adsorption non spécifique est entravée et dans lequel aucune étape de blocage de protéines n'est effectuée.

25. Kit selon les revendications 18 à 23, dans lequel le colloïde recouvert de la SAM est stable dans un liquide biologiquement pertinent sans détergents.

26. Kit selon les revendications 18 à 23, dans lequel le colloïde recouvert de la SAM est formé par le fait de comprendre l'utilisation de thiols à terminaison triéthylèneglycol qui sont mélangés dans la solution de thiols pour empêcher une liaison non spécifique.

27. Kit selon les revendications 18 à 23, dans lequel le colloïde recouvert de la SAM est formé par le fait de comprendre l'exposition de colloïdes à des espèces moléculaires formant une monocouche autoassemblée et un carboxylate et/ou un tensioactif.
